# EUROPEAN PATENT SPECIFICATION

(11) **EP 1 581 559 B1**
(45) Date of publication and mention of the grant of the patent: **30.06.2010**
(21) Application number: 03786140.8
(22) Date of filing: 24.12.2003
(51) Int. Cl.: C07K 16/00, C07K 16/40, C07K 16/24

(54) **VL DAb FC FUSION**
VL DAb FC-FUSION
FUSION DE VL DAb AVEC FC

(30) Priority: 27.12.2002 GB 0230203
(43) Date of publication of application: 05.10.2005
(60) Divisional application: 07001932.8
(73) Proprietor: Domantis Limited, Cambridge Cambridgeshire, CB4 OWG (GB)
(72) Inventor: WINTER, Greg, Cambridge CB2 1TQ (GB); TOMLINSON, Ian, Cambridge Cambridgeshire CB4 OWG (GB); IGNATOVICH, Olga, Cambridge Cambridgeshire CB4 OWG (GB); BREWIS, Neil, Cambridge Cambridgeshire CB4 OWG (GB)
(74) Representative: Furlong, Isla Jane
(86) International application number: PCT/GB2003/005597
(87) International publication number: WO 2004/058820

(56) References cited:
- EP-A- 0 368 684
- EP-A1- 0 318 554
- WO-A-02/44215
- WO-A-94/04678
- WO-A-02/085945
- WO-A1-98/20140
- US-A- 3 907 502
- US-A- 5 659 013
- US-A- 5 869 046
- US-B1- 6 248 516
- D. POWERS ET AL.: "Expression of single-chain Fv-Fc fusions in Pichia pastoris." JOURNAL OF IMMUNOLOGICAL METHODS, vol. 251, 2001, pages 123-135, XP004233079 Amsterdam, The Netherlands
- L. RIECHMANN ET AL.: "Single domain antibodies: comparison of camel VH and camelised human VH domains." JOURNAL OF IMMUNOLOGICAL METHODS, vol. 231, 1999, pages 25-38, XP004187632 Amsterdam, The Netherlands
- SOLOMON A. ET AL: 'Light chain-associated amyloid deposits comprised of a novel kappa constant domain.' PROCEEDINGS OF THE NATIONAL ACADEMY OF SCIENCES OF THE UNITED STATES OF AMERICA 4 AUG 1998 vol. 95, no. 16, 04 August 1998, pages 9547 - 9551 ISSN: 0027-8424

## Description

The present invention relates to a simple method for generating antibody molecules suitable for *in vivo* use. In particular, the invention relates to a method for the generation of antibody molecules suitable for *in vivo* use which are based on antibody single variable domains.

### Introduction

The antigen binding domain of an antibody comprises two separate regions: a heavy chain variable domain (V_{H}) and a light chain variable domain (V_{L}: which can be either V_{κ}Vₖ or V_{λ}). The antigen binding site itself is formed by six polypeptide loops: three from V_{H} domain (H1, H2 and H3) and three from V_{L} domain (L1, L2 and L3). A diverse primary repertoire of V genes that encode the V_{H} and V_{L} domains is produced by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) Immunol Today, 16: 237), 25 functional D segments (Corbett et al. (1997) J. Mol. Biol., 268: 69) and 6 functional J_{H} segments (Ravetch et al. (1981) Cell, 27: 583), depending on the haplotype. The V_{H} segment encodes the region of the polypeptide chain which forms the first and second antigen binding loops of the V_{H} domain (H1 and H2), whilst the V_{H}, D and J_{H} segments combine to form the third antigen binding loop of the V_{H} domain (H3). The V_{L} gene is produced by the recombination of only two gene segments, V_{L} and J_{L}. In humans, there are approximately 40 functional V_{κ} segments (Schäble and Zachau (1993) Biol. Chem. Hoppe-Seyler, 374: 1001), 31 functional V_{λ} segments (Williams et al. (1996) J. Mol. Biol., 264: 220; Kawasaki et al. (1997) Genome Res., 7: 250), 5 functional J_{κ} segments (Hieter et al. (1982) J. Biol. Chem., 257: 1516) and 4 functional J_{λ} segments (Vasicek and Leder (1990) J. Exp. Med., 172: 609), depending on the haplotype. The V_{L} segment encodes the region of the polypeptide chain which forms the first and second antigen binding loops of the V_{L} domain (L1 and L2), whilst the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) J. Mol. Biol., 196: 901; Chothia et al. (1989) Nature, 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences *(*Chothia et al. (1992) J. Mol. Biol., 227: 799; Tomlinson et al. (1995) EMBO J., 14: 4628; William el al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1.

Historically, antibodies have been obtained from natural sources such as by the immunisation of rabbits and other such animals. Alternatively, molecular biology techniques may be employed and antibodies may be generated using techniques such as those involving the use of hybrid hybridomas.

In this way antibodies of a selected or desired antigen binding specificity can be generated. Such antibodies are of great therapeutic value as they can be designed against disease antigens, for instance. However, the method of production of these antibodies is laborious and prone to error, as well as being limited to diversity resulting from the immunisation history of the donor. It would be an advantage to generate increased diversity, e.g. using synthetic libraries. Therefore, there remains in the art a need for a simple method of generating functionally active antibody molecules of a desired or predetermined antigen binding specificity.

Single heavy chain variable domains have been described, derived from natural antibodies which normally comprise light chains, from monoclonal antibodies or from repertoires of domains (EP-A-0368684). These heavy chain variable domains have been shown to interact specifically with one or more antigens (Ward *et al*,). However, these single domains have been shown to have a very short *in vivo* half-life. Therefore such domains are of limited therapeutic value.

In addition, EP 0 656 946A1 describes dual-chain immunoglobulin molecules which bind antigen specifically, and in which the heavy polypeptide chains are devoid of CH1 heavy chain domains, the immunoglobulin also being devoid of light polypeptide chains. Such antibodies are naturally occurring in Camelids, and therefore, as such the antigen specificity of the antibody is limited to those generated by the Camelid.

Also noteworthy are studies performed on Heavy Chain Disease. In this disease immunoglobulin molecules are generated which comprise a heavy chain variable domain, CH2 and CH3 domains, but lack a CH1 domain and light chains. Such molecules are found to accumulate in Heavy Chain Disease (Block et al, Am J. Med, 55,61-70 (1973), Ellman et al, New Engl. J. Med, 278: 95-1201 (1968) ). Thus, the Heavy Chain Disease prior art teaches that antibodies comprising a single antigen interaction domain type only (in this case heavy chain variable domains) are associated with disease. That is, the prior art teaches away from the use of antibodies based solely on human heavy chain variable domains for prophylactic and/or therapeutic use.

International patent application W088/09344 (Creative Biomolecules) describes antibody constructs comprising linkers to link domains.

EP-A-0368684 describes a single domain ligand consisting of at least part of the variable domain of one chain of a molecule from the immunoglobulin superfamily.

US 6,248,516 relates to a library for expression of immunoglobulin heavy chain variable domains (VH domains) which library comprises a repertoire of nucleic acid sequences encoding a third CDR of an immunoglobulin heavy chain variable domain.

WO 02/085945 describes a method for the production of a VHH single heavy chain antibody in a mammal comprising the step of expressing a heterologous VHH heavy chain locus in that mammal.

WO 94/04678 describes an immunoglobulin comprising two heavy polypeptide chains sufficient for the formulation of a complete antigen binding site or several antigen binding sites, the immunoglobulin being further devoid of light polypeptide chains.

US 5,869,046 describes a method for preparing a variant Fab or (Fab')₂ which contains an Ig domain or Ig-like domain comprising at least one of a CH1 or C_{L} region that is cleared from the kidney and does not contain a Fc region of an IgG.

WO 02/44215 describes a binding molecule comprising (i) one or more polypeptides which form a binding site capable of binding a target molecule and (ii) Fc effector peptide displaying one or more effector functions associated with the constant region (Fc) of an immunoglobulin heavy chain.

Powers et al (2001) (J. Immunol. Methods 251: 123-135) describes the expression of single-chain Fv - Fc fusions in *Pichia pastoris*.

Riechmann and Muldermans (1999) (J. Immunol. Methods 231:25-38) discuss single domain antibodies and compare camel VH and camelised human VH domains.

Soloman et al (1998) (PNAS. 95: 9547 - 9551) describes light chain-associated amyloid deposits comprised of a novel κ constant domain.

US 3,907,502 describes a method for the detection, typing and quantitative evaluation of Bence Jones proteins in biological fluids.

WO 98/20140 relates to a composition comprising a substantially purified protein having tumour necrosis factor receptor (TNF-R) releasing enzymatic activity, the protein having a native form which exhibits an apparent molecular weight of about 120kD.

Therefore, there remains a need in the art for a simple and non-laborious method for the generation of antibody based molecules of a desired or predetermined antigen binding specificity not necessarily limited by the pre-exposure of the donor to antigen which are suitable for prophylactic and/or therapeutic use.

### Summary of the invention

The present inventors have devised a simple and non-laborious method for the synthesis of antibody based molecules of a selected epitope binding specificity, which are suitable for *in vivo* prophylactic and/or therapeutic use. Significantly, the method of the invention permits the synthesis of single chain antibody based molecules of a desired or predetermined epitope binding specificity. The use of this simple method is surprising in light of the Heavy Chain disease prior art which teaches away from the therapeutic use of heavy chain-only antibodies.

Structurally, the molecules of the present invention comprise an antibody single variable domain having a defined or predetermined epitope binding specificity and one or more antibody constant regions and/or hinge region (collectively termed ("an effector group"). Such a molecule is referred to as a single domain-effector group immunoglobulin (dAb-effector group) and the present inventors consider that such a molecule will be of considerable therapeutic value.

Thus, in a first aspect, the present invention provides a method for synthesising a single-domain-effector group immunoglobulin (dAb-effector group) suitable for *in vivo* use comprising the steps of:
(a) selecting an antibody single variable domain having an epitope binding specificity; and
(b) attaching the single domain of step (a) to an immunoglobulin effector group
wherein the antibody single variable domain is a light chain variable domain.

According to the present invention, preferably the antibody single domain is a non-camelid antibody single domain. Advantageously, it is single variable of human origin.

In another embodiment, the single variable domain comprises non-Camelid (eg, human) framework regions (eg, 1, 2, 3 or 4 human framework regions). Advantageously one or more of the human framework regions (as defined by Kabat) are identical on the amino acid level to those encoded by human germline antibody genes.

Variable region sequences in, for example, the Kabat database of sequences of immunological interest, or other antibody sequences known or identifiable by those of skill in the art can be used to generate a dAb-effector group as described herein. The Kabat database or other such databases include antibody sequences from numerous species.

CDRs and framework regions are those regions of an immunoglobulin variable domain as defined in the Kabat database of Sequences of Proteins of Immunological Interest. Preferred human framework regions are those encoded by germline gene segment DPK9. Advantageously, FW1, FW2 and FW3 of a V_{L} domain have the sequence of FW1, FW2 or FW3 from DPK9. The human frameworks may optionally contain mutations, for example up to about 5 amino acid changes or up to about 10 amino acid changes collectively in the human frameworks used in the ligands of the invention.

Advantageously, the antibody single variable domains used according to the methods of the present invention are isolated, at least in part by human immunisation. Advantageously they are not isolated by animal immunisation.

In one embodiment, the single variable domain comprises a binding site for a generic ligand as defined in WO 99/20749. For example, the generic ligand is Protein L.

As herein defined, the term 'single-domain-effector group immunoglobulin molecule' (dAb-effector group) describes an engineered immunoglobulin molecule comprising a single variable domain capable of specifically binding one or more epitopes, attached to one or more constant region domains and/or hinge (collectively termed "an effector group"). The variable domain is a light chain domain (V_{L}). Each light chain domain may be either of the kappa or lambda subgroup. Advantageously, an effector group as herein described comprises an Fc region of an antibody.

dAb-effector groups may be combined to form multivalent structures, including any of those selected from the group consisting of the following: homodimers, heterodimers and multimers. Such multimeric structures have improved avidity of antigen interaction by virtue of the multimeric structures having more than one epitope binding site where the epitopes are on the same antigen. Where the epitopes are on different antigens, eg those close together on the same cell surface, these epitopes may be bridged by dAb-effector groups.

For the avoidance of doubt, dAb-effector groups according to the invention do not include the dual-chain antibodies as described in EP-A-0656946 as well as single chain fragments disclosed therein, such as V_{HH}-hinge fragments, based on camelid immunoglobulins. In addition, the term 'dAb-effector group' does not include within its scope the naturally occurring dual chain antibodies generated within Camelids. Nor does the term 'dAb-effector group' include within its scope the four-chain structure of IgG antibody molecules comprising two light and two heavy chains or single heavy or light chains derived therefrom.

As referred to above, the term 'suitable for *in vivo* use' means that the 'dAb-effector group' according to the present invention has sufficient half-life such that the molecule is present within the body for sufficient time to produce one or more desired biological effects. In this regard the present inventors have found that the size and nature of the effector group influences the half-life of the dAb-effector groups according to the invention.

A preferred effector group according to the present invention is or comprises the Fc region of an antibody molecule. Such an effector group permits Fc receptor binding (e.g. to one or both of Fc receptors CD64 and CD32) and complement activation via the interaction with C1q, whilst at the same time providing the molecule with a longer half-life than a single variable heavy chain domain in isolation.

As used herein, the term 'epitope' is a unit of structure conventionally bound by an antigen binding site as provided by one or more variable domains, e.g. an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation of any other variable domain.

As used herein, the term 'select' (an antibody variable domain) includes within its scope the selection of (an antibody variable domain) from a number of different alternatives. Techniques for the 'selection' of antibody variable domains will be familiar to those skilled in the art. The term 'selection' (of an antibody variable domain) includes within its scope the 'selection' of one or more variable domains by library screening. Advantageously, the selection involves the screening of a repertoire of antibody variable domains displayed on the surfaces of bacteriophage within a phage display library (McCafferty et al, (1990) Nature 340, 662-654) or emulsion-based *in vitro* systems (Tawfik & Griffiths (1998) Nature Biotechnol 16(7), 652-6).

As used herein the term 'attaching' (the single domain as herein described to an effector group) includes within its scope the direct attachment of a single domain as described herein to one or more constant regions as herein described. It also includes the indirect attachment of a single domain to an effector group via for example a further group and/or a linker region. Furthermore, the term 'attaching' includes within its scope an association of the respective groups such that the association is maintained *in vivo* such that the dAb-effector group is capable of producing biological effects, such as increasing half life (i.e., serum residence time of the variable domain) and allowing the functional attributes of, for example, constant regions, such as Fc regions, to be exploited *in vivo*.

In a preferred embodiment, the variable domain and the effector group are directly attached, without the use of a linker.

In the case that a linker is used to attach a variable domain to one or more constant region domains, the linker is advantageously a polypeptide linker. One skilled in the art will appreciate that the length and composition of the linker may affect the physical characteristics of the dAb-effector group. Thus, a short linker may minimise the degree of freedom of movement exhibited by each group relative to one another, whereas a longer linker may allow more freedom of movement. Likewise bulky or charged amino acids may also restrict the movement of one domain relative to the other. Discussion of suitable linkers is provided in Bird et al. Science 242, 423-426. Hudson et al, Journal Immunol Methods 231 (1999) 177-189; Hudson et al, Proc Nat Acad Sci USA 85, 5879-5883. One example is a (Gly₄ Ser)ₙ linker, where n=l to 8, eg, 2, 3 or 4.

The attachment of a single variable domain to an effector group, as herein defined may be achieved at the polypeptide level, that is after expression of the nucleic acid encoding the respective domains and groups. Alternatively, the attachment step may be performed at the nucleic acid level. Methods of attachment an include the use of protein chemistry and/or molecular biology techniques which will be familiar to those skilled in the art and are described herein.

As defined herein the term 'non-camelid antibody single variable domain' refers to an antibody single variable domain of non-camelid origin. The non-camelid antibody single variable domain may be selected from a repertoire of single domains, for example from those represented in a phage display library. Alternatively, they may be derived from native antibody molecules. Those skilled in the art will be aware of further sources of antibody single variable domains of non-camelid origin.

Antibody single variable domains are light chain variable domains (V_{L}). Each V_{L} chain variable domain is of the Vkappa (Vκ) or Vlambda (Vλ) sub-group.

According to one embodiment of the present invention, an antibody variable domain (V_{L}) is attached to one or more antibody constant region heavy domains. Such one or more constant heavy chain domains constitute an 'effector group' according to the present invention.

In one embodiment, each V_{L} domain is attached to an Fc region (an effector group) of an antibody. Advantageously, a dAb-effector group according to the invention is V_{L}- Fc. In the case that the effector group is an Fc region of an antibody, then the CH3 domain facilitates the interaction of a dAb-effector group with Fc receptors whilst the CH2 domain permits the interaction of a dAb-effector group with Clq, thus facilitating the activation of the complement system. In addition, the present inventors have found that the Fc portion of the antibody stabilises the dAb-effector group and provides the molecule with a suitable half-life for *in vivo* therapeutic and/or prophylactic use.

Other suitable effector groups include any of those selected from the group consisting of the following: an effector group comprising at least an antibody light chain constant region (CL), an antibody CH1 heavy chain domain, an antibody CH2 heavy chain domain, an antibody CH3 heavy chain domain, or any combination thereof. In addition to the one or more constant region domains, an effector group may also comprise a hinge region of an antibody (such a region normally being found between the CH1 and CH2 domains of an IgG molecule). In a further embodiment of the above aspect of the invention, the effector group is a hinge region alone such that the dAb-effector group comprises a single variable domain attached to the hinge region of an antibody molecule.

According to the present invention, advantageously an effector group as herein described is or comprises the constant region domains CH2 and/or CH3. Advantageously, the effector group-comprises CH2 and/or CH3, preferably an effector group as herein described consists of CH2 and CH3 domains, optionally attached to a hinge region of an antibody molecule as described herein.

In a further aspect, the present invention provides a 'dAb-effector group' obtainable using the methods of the present invention. For the avoidance of any doubt, 'dAb-effector groups' according to the present invention, do not include within their scope the four chain structure of IgG molecules nor the dual-chain structure of naturally occurring Camelid antibodies or those described in EP 0 656 946 A1.

Antibody single variable domains are light chain variable domains (V_{L}). Each V_{L} chain variable domain is of the Vkappa (Vκ) or Vlambda (Vλ) sub-group. The use of V_{L} domains has the advantage that these domains unlike variable heavy chain domains (V_{H}) do not possess hydrophobic interfaces which are 'sticky' and can cause solubility problems in the case of isolated V_{H} domains.

Structurally, single domain effector group immunoglobulin molecules according to the present invention comprise V_{L} domains as described above.

According to the above aspect of the invention, advantageously the dAb-effector group obtained by the methods of the invention is an V_{H}-Fc or a V_{L}-Fc. More advantageously, the dAb-effector group is V_{L}-Fc. In an alternative embodiment of this aspect of the invention the dAb-effector group is V_{H}-hinge. In an alternative embodiment still, the dAb-effector group is a Vk-Fc. The present inventors have found that the Fc portion of the antibody stabilises the dAb-effector group providing the molecule with a suitable half-life.

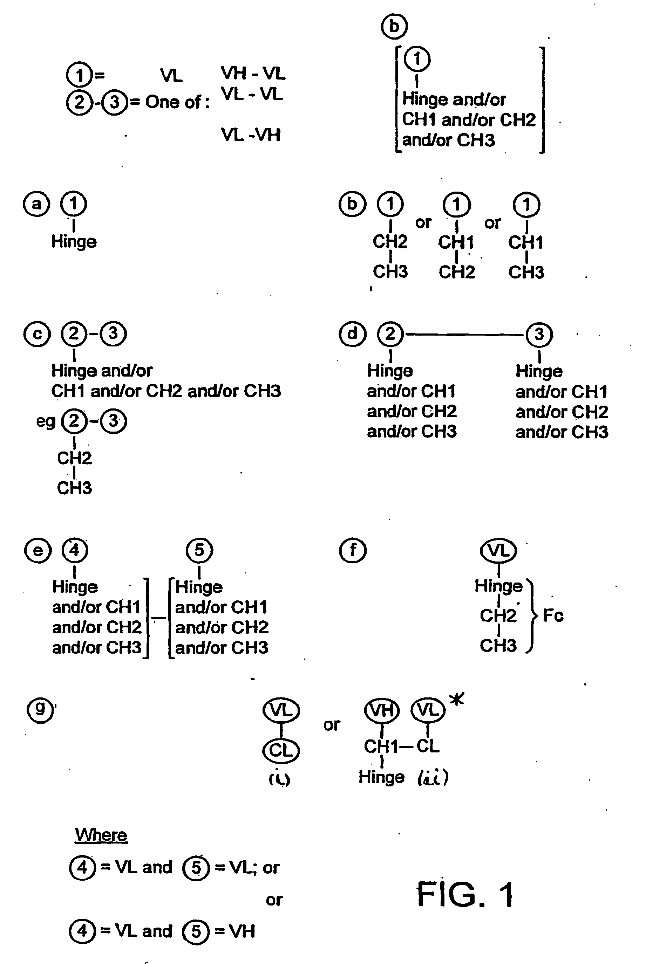

In an alternative embodiment of this aspect of the invention, the effector group is based on a Fab antibody fragment. That is, it comprises an antibody fragment comprising a V_{H} domain or a V_{L} domain attached to one or more constant region domains making up a Fab fragment. One skilled in the art will appreciate that such a fragment comprises only one variable domain. Such Fab effector-groups are illustrated in Fig 1.

Various preferred 'dAb-effector groups' prepared according to the methods of the present invention are illustrated in Fig 1.

The dAb-effector groups of the present invention may be combined onto non-immunoglobulin multi-ligand structures so that they form multivalent structures comprising more than one antigen binding site. Such structures have an increased avidity of antigen binding. In an example of such multimers, the V regions bind different epitopes on the same antigen providing superior avidity. In another embodiment multivalent complexes may be constructed on scaffold proteins, as described in WO0069907 (Medical Research Council), which are based for example on the ring structure of bacterial GroEL or on other chaperone polypeptides.

Alternatively, dAb-effector groups according to the present invention, may be combined in the absence of a non-immunoglobulin protein scaffold to form multivalent structures which are solely based on immunoglobulin domains. Such multivalent structures may have increased avidity towards target molecules, by virtue of them comprising multiple epitope binding sites. Such multivalent structures may be homodimers, heterodimers or multimers.

The present inventors consider that dAb-effector groups of the invention, as well as such multivalent structures, will be of particular use for use in prophylactic and/or therapeutic uses.

Antigens may be, or be part of, polypeptides, proteins or nucleic acids, which may be naturally occurring or synthetic. One skilled in the art will appreciate that the choice is large and varied. They may be for instance human or animal proteins, cytokines, cytokine receptors, enzymes co-factors for enzymes or DNA binding proteins. Suitable cytokines and growth factors include but are not limited to: ApoE, Apo-SAA, BDNF, Cardiotrophin-1, EGF, EGF receptor, ENA-78, Eotaxin, Eotaxin-2, Exodus-2, EpoR, FGF-acidic, FGF-basic, fibroblast growth factor-10, FLT3 ligand, Fractalkine (CX3C), GDNF, G-CSF, GM-CSF, GF-β1, insulin, IL1R1, IFN-γ, IGF-I, IGF-II, IL-1α, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein (30 *ibid*), M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, p55, TNFα recognition site, pro-TNF-α-stalk, PDGF-AA, PDGF-AB, PDGF-BB, PF-4, RANTES, SDF1α, SDF1β, SCF, SCGF, stem cell factor (SCF), TARC, TACE enzyme recognition site, TGF-α, TGF-β, TGFβ1, TGF-β2, TGF-β3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-γ, HCCl, 1-309, HER 1, HER 2, HER 3 and HER 4. Cytokine receptors include receptors for the foregoing cytokines. It will be appreciated that this list is not intended to be exhaustive.

In one embodiment of the invention, the variable domains are derived from an antibody directed against one or more antigen/s or epitope/s. In this respect, the dAb-effector group of the invention may bind the epiotpe/s or antigen/s and act as an antagonist or agonist (eg, EPO receptor agonist).

In a preferred embodiment the variable domains are derived from a repertoire of single variable antibody domains. In one example, the repertoire is a repertoire that is not created in an animal or a synthetic repertoire. In another example, the single variable domains are not isolated (at least in part) by animal immunisation. Thus, the single domains can be isolated from a naïve library.

In one aspect, a library (eg, phage or phagemid library or using emulsion technology as described in WO 99/02671) is made wherein a population of library members each comprises a common construct encoding an effector group (eg, an Fc region). A diversity of sequences encoding single variable domains is then spliced in to form a library of members displaying a diversity of single variable domains in the context of the same effector group. dAb-effector group selection against antigen or epitope is then effected in the context of the common effector group, which may have been selected in the basis of its desirable effects on half life, for example.

In a further aspect, the present invention provides one or more nucleic acid molecules encoding at least a dAb-effector group as herein defined.

The dAb-effector group may be encoded on a single nucleic acid molecule; alternatively, different parts of the molecule may be encoded by separate nucleic acid molecules. Where the 'dAb-effector group' is encoded by a single nucleic acid molecule, the domains may be expressed as a fusion polypeptide, or may be separately expressed and subsequently linked together, for example using chemical linking agents. dAb-effector groups expressed from separate nucleic acids will be linked together by appropriate means.

The nucleic acid may further encode a signal sequence for export of the polypeptides from a host cell upon expression and may be fused with a surface component (eg, at least part of the pIII coat protein) of a filamentous bacteriophage particle (or other component of a selection display system) upon expression.

In a further aspect the present invention provides a vector comprising nucleic acid according to the present invention.

In a yet further aspect, the present invention provides a host cell transfected with a vector according to the present invention.

Expression from such a vector may be configured to produce, for example on the surface of a bacteriophage particle, dAb-effector groups for selection.

The dAb-effector group according to the present invention may be provided in the form of a kit suitable for the prophylaxis and/or treatment of disease.

In a further aspect still, the present invention provides a composition comprising a dAb-effector group, according to the first aspect of the present invention, and a pharmaceutically acceptable carrier, diluent or excipient.

As discussed previously, the present inventors have found that the size and nature of the effector group enhances the half-life of a dAb-effector group according to the present invention. Methods for pharmacokinetic analysis will be familiar to those skilled in the art Details may be found in Kenneth, A et al: Chemical Stability of Pharmaceuticals: A Handbook for Pharmacists and in Peters et al, Pharmacokinetic analysis: A Practical Approach (1996). Reference is also made to "Pharmacokinetics", M Gibaldi & D Perron, published by Marcel Dekker, 2nd Rev. ex edition (1982), which describes pharmacokinetic parameters such as t alpha and t beta half lives and area under the curve (AUC).

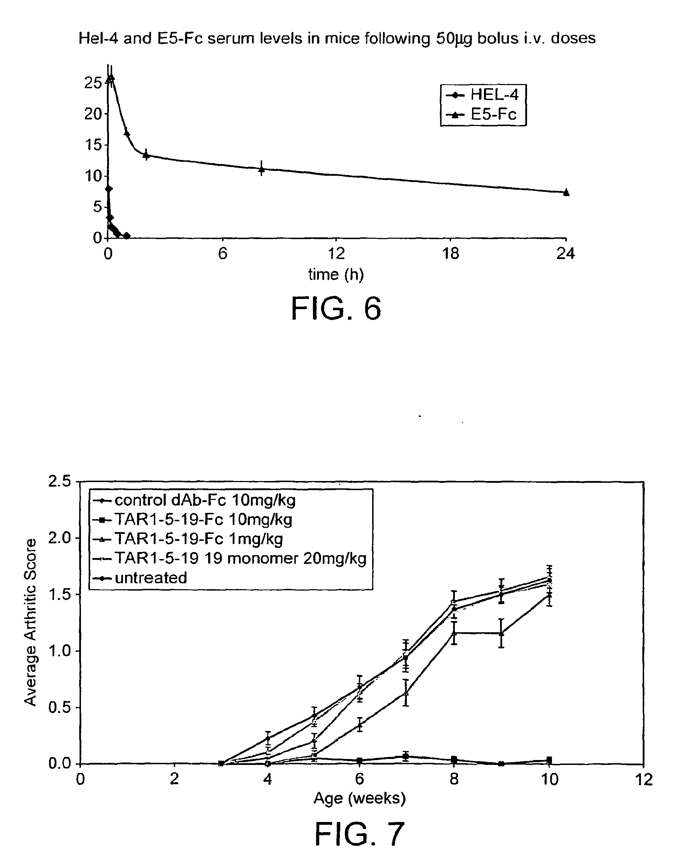

Half lives (t½ alpha and t½ beta) and AUC can be determined from a curve of serum concentration of dAb-Effector Group against time (see, eg figure 6). The WinNonlin analysis package (available from Pharsight Corp., Mountain View, CA94040, USA) can be used, for example, to model the curve. In a first phase (the alpha phase) the dAb-Effector Group is undergoing mainly distribution in the patient, with some elimination. A second phase (beta phase) is the terminal phase when the dAb-Effector Group has been distributed and the serum concentration is decreasing as the dAb-Effector Group is cleared from the patient. The t alpha half life is the half life of the first phase and the t beta half life is the half life of the second phase.

Thus, advantageously, the present invention provides a dAb-effector group or a composition comprising a dAb-effector group according to the invention having a tβ half-life in the range of 15 minutes or more. In one embodiment, the lower end of the range is 30 minutes, 45 minutes, 1 hour, 2 hours, 3 hours, 4 hours, 5 hours, 6 hours, 7 hours, 10 hours, 11 hours or 12 hours. In addition, or alternatively, a dAb-effector group or composition according to the invention will have a tβ half-life in the range of up to and including 12 hours. In one embodiment, the upper end of the range is 11, 10, 9, 8, 7, 6 or 5 hours. An example of a suitable range is 1 to 6 hours, 2 to 5 hours or 3 to 4 hours.

Advantageously, the present invention provides a dAb-effector group or a composition comprising a dAb-effector group according to the invention having a tβ half-life in the range of 2.5 hours or more. In one embodiment, the lower end of the range is 3 hours, 4 hours, 5 hours, 6 hours, 7 hours, 10 hours, 11 hours, or 12 hours. In addition, or alternatively, a dAb-effector group or composition according to the invention has a tβ half-life in the range of up to and including 21 days. In one embodiment, a dAb-effector group according to the invention has a tβ half-life of any of those tβ half-lifes selected from the group consisting of the following: 12 hours or more, 24 hours or more, 2 days or more, 3 days or more, 4 days or more, 5 days or more, 6 days or more, 7 days or more, 8 days or more, 9 days or more, 10 days or more, 11 days or more, 12 days or more, 13 days or more, 14 days or more, 15 days or more or 20 days or more. Advantageously a dAb-effector group or composition according to the invention will have a tβ half-life in the range 12 to 60 hours. In a further embodiment, it will have a tβ half-life or a day or more. In a further embodiment still, it will be in the range 12 to 26 hours.

Advantageously, a dAb-effector group according to the present invention comprises or consists or a V_{L}-Fc having a tβ half-life of a day or more, two days or more, 3 days or more, 4 days or more, 5 days or more, 6 days or more, 7 days or more. Most advantageously, a dAb-effector group according to the present invention comprises or consists of an V_{L}-Fc having a tβ half-life of a day or more.

According to the present invention, more advantageously, a dAb-effector group according to the present invention comprises an effector group consisting of the constant region domains CH2 and/or CH3, preferably CH2 and CH3, either with or without a hinge region as described herein, wherein the dAb-effector group has a tβ half-life of a day or more, two days or more, 3 days or more, 4 days or more, 5 days or more, 6 days or more, 7 days or more. Most advantageously, a dAb-effector group according to the present invention comprises an effector group consisting of the constant region domains CH2 and/or CH3 wherein the dAb-effector group has a tβ half-life of a day or more.
In addition, or alternatively to the above criteria, the present invention provides a dAb-effector group or a composition comprising a dAb-effector group according to the invention having an AUC value(area under the curve) in the range of 1 mg.min/ml or more. In one embodiment, the lower end of the range is 5, 10, 15, 20, 30, 100, 200 or 300mg.min/ml. In addition, or alternatively, a dAb-effector group or composition according to the invention has an AUC in the range of up to 600 mg.min/ml. In one embodiment, the upper end of the range is 500, 400, 300, 200, 150, 100, 75 or 50 mg.min/ml. Advantageously a dAb-effector group according to the invention will have a AUC in the range selected from the group consisting of the following: 15 to 150mg.min/ml, 15 to 100 mg.min/ml, 15 to 75 mg.min/ml, and 15 to 50mg.min/ml.

In a further aspect, the present invention provides the use of a dAb-effector group or a composition according to the present invention in the preparation of a medicament for the prophylaxis and/or treatment of disease.

In a further aspect, the present invention provides a dAb-effector group according to the present invention or a composition thereof in the treatment of disease.

Furthermore, the present inventors have found that a dAb-Fc specific for target human TNF alpha and designated TARI-5-19-Fc was shown to be a highly effective therapy in a model of arthritis. Thus the inventors consider that a TAR1-5-19-effector group may be of particular use in the prophylaxis and/or treatment of one or more inflammatory diseases.

In a further aspect the present invention provides the use of a dAb-effector group according to the invention in the preparation of a medicament for the prophylaxis and/or treatment of one or more inflammatory diseases.

According to the above aspects of the invention, advantageously, the dAb-effector group specifically binds to TNF alpha. More advantageously, the dAb-effector group specifically binds to human TNF alpha. More advantageously still, the dAb-effector group is a dAb-Fc and specifically binds to TNF alpha, preferably human TNFalpha. -More advantageously still, the dAb-effector group comprises TAR1-5-19 as effector group. Most advantageously, the dAb-effector group for use according to the above aspects of the invention is TAR1-5-19-Fc.

According to the above aspects of the invention, advantageously, the one or more inflammatory diseases are mediated by TNF-alpha. Advantageously, the one or more inflammatory diseases are mediated by TNFalpha and are selected from the group consisting of the following: rheumatoid arthritis, psoriasis, Crohns disease, inflammatory bowel disease (IBD), multiple sclerosis, septic shock, alzheimers, coronary thrombosis, chronic obstructive pulmonary disease (COPD) and glomerular nephritis.

In a further aspect still the invention provides the use of a dAb-effector group according to the invention in the preparation of a medicament for reducing and/or preventing and/or suppressing cachexia in patient.

In a preferred embodiment of the above aspects of the invention, the cachexia is associated with an inflammatory disease. Advantageously, the inflammatory disease is selected from the group consisting of the following: rheumatoid arthritis, psoriasis, Crohns disease, inflammatory bowel disease (IBD), multiple sclerosis, septic shock, alzheimers, coronary thrombosis, chronic obstructive pulmonary disease (COPD) and glomerular nephritis.

According to the above aspects of the invention, the use may be used for the treatment of human or non-human patients. According to the above aspects of the invention, preferably, the patient is a human and the TNFalpha is human TNFalpha.

Suitable dosages for the administration to a subject of dAb-effector group according to the invention will be familiar to those skilled in the art. Advantageously, the dose is in the range of between 0.5 to 20mg/Kg of dAb-effector group. More advantageously, the dosage of dAb-effector group is in the range of between 1 to 10mg/Kg. Preferred dosage ranges are between 1 and 5 mg/Kg. Most advantageously, dosages of 1mg/Kg or 5mg/Kg dAb-effector group are administered. Suitable dosage regimes may be dependent upon certain subject characteristics including age, severity of disease and so on. dAb-effector group may for example be administered, particularly when the subject is a human, daily, once a week, twice a week or monthly. Those skilled in the art will appreciate that this list is not intended to be exhaustive.

### Brief Description of the Figures.

**Figure 1** shows various preferred dAb-effector groups according to the present invention.
   (a) shows V_{L} attached to the hinge region of an antibody molecule.
   (b) shows V_{L} attached to CH1 or CH2 or CH3.
   (c) shows V_{L} and V_{L} or V_{H} and attached to CH and CH2 or CH3
   (d) shows a dAB-effector group according to (b) attached to V_{H} or V_{L}
   (e) shows a dimer of V_{L} and V_{L} or V_{H} attached to any combination of CH1/CH2 and CH3 domains, in which the variable domains are attached to one another, cither with or without the use of a linker as herein described.
   (f) Shows a dimer having the same components as step (e) but in which the point of attachment between the two components making up the dimer is the effector groups.
   (g) Shows V_{L} attached to the Fc region of an antibody molecule.
   * the single variable domains V_{H} and V_{L} each form a respective epitope or antigen binding site and the epitope or antigen specificity of the single variable domains is different.
**Figure 2** shows the signal pIgplus vector used to create E5-Fc and VH2-Fc fusions. Details are given in Example 1.

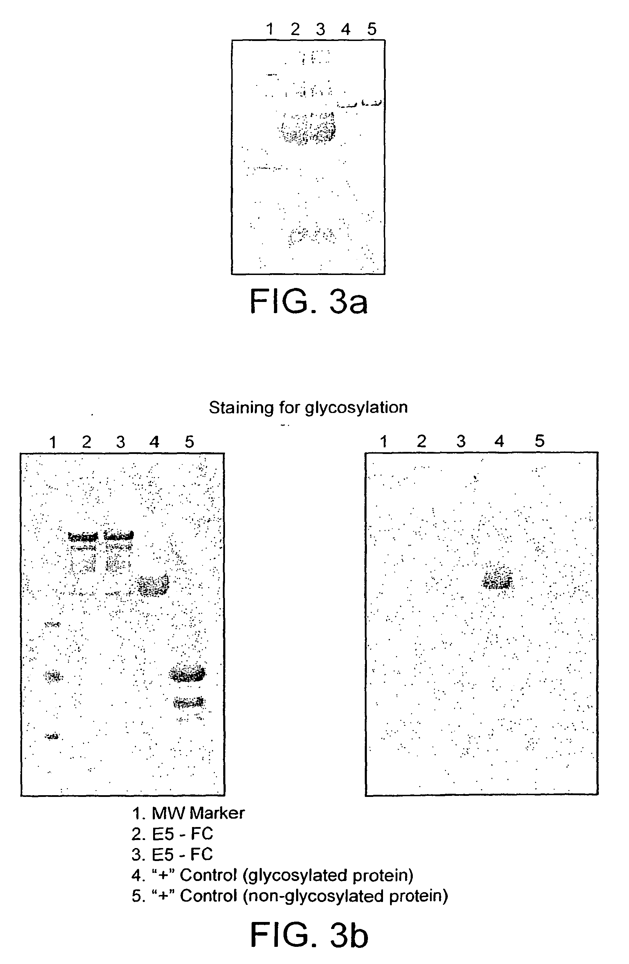

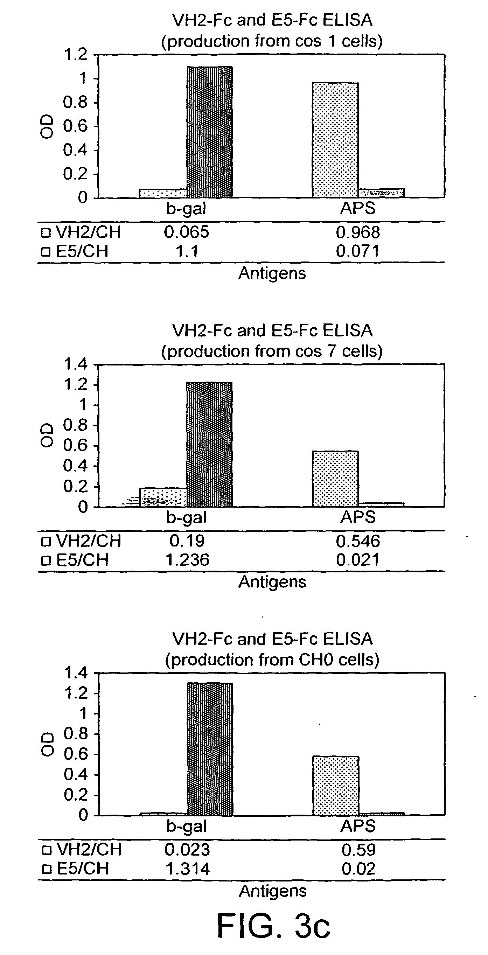

**Figure 3a** shows SDS page gels representing the purification of immunoglobulin effector groups according to the invention; Lane I-MW marker (kDa), Lane 2-Culture medium before Protein A purification, Lane 3-Culture medium after Protein A purification, Lane 4-Purified E5-Fc protein, Lane 5-Purified E5-Fc protein. Figure 3b shows the glycosylation of immunoglobulin-effector groups according to the invention. Lanes are labelled on the figure. Figure 3c shows ELISA results demonstrating that Cos-1 cells, Cos-7 cells and CHO cells are capable of expressing dAb-Fc fusion proteins of the correct specificity and with no cross reactivity with irrelevant antigens.

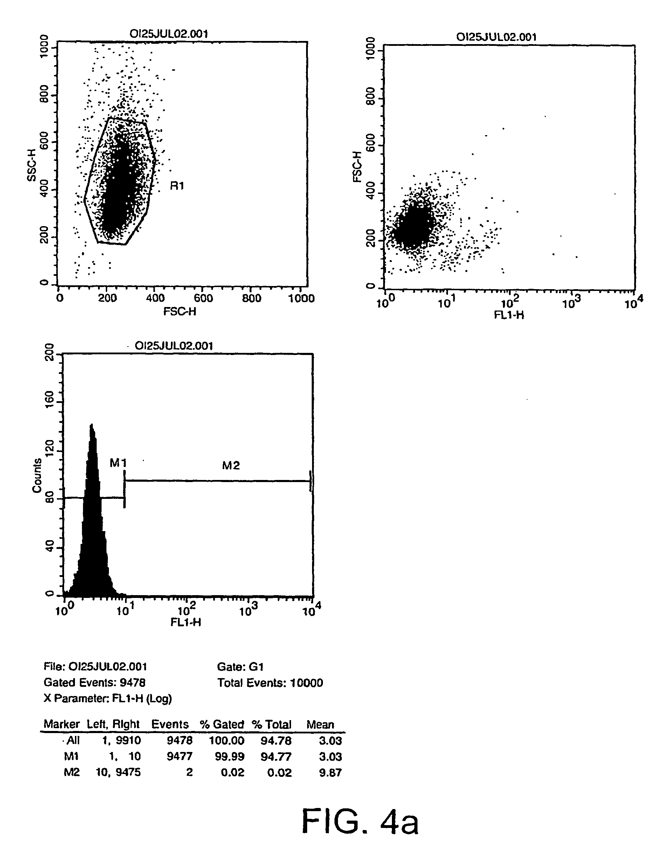

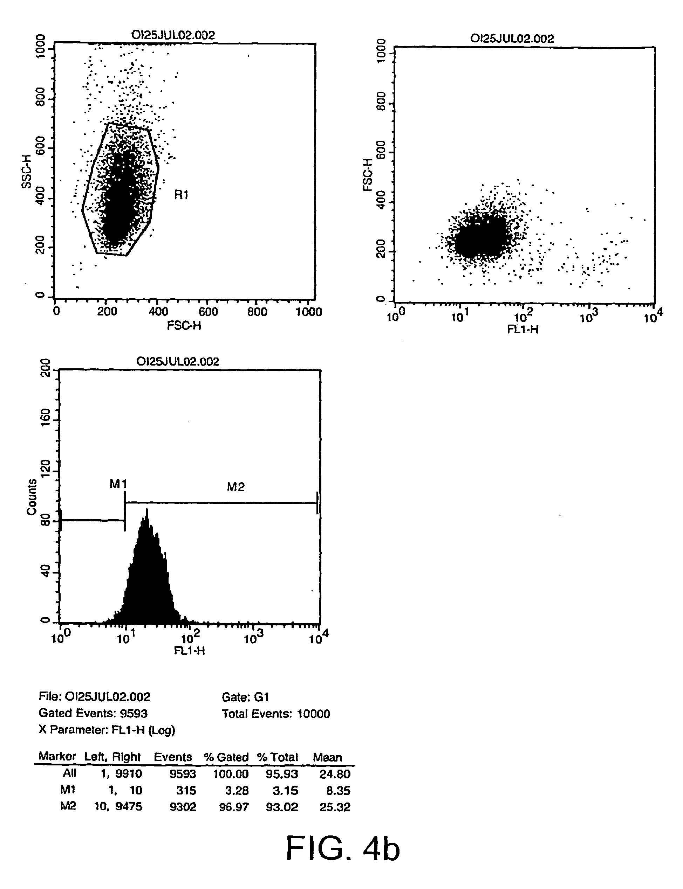

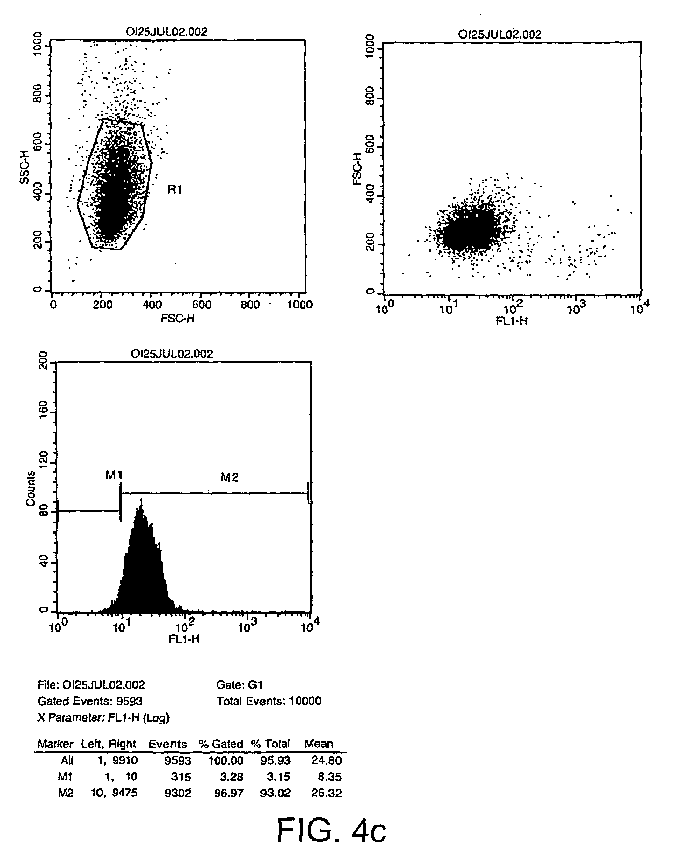

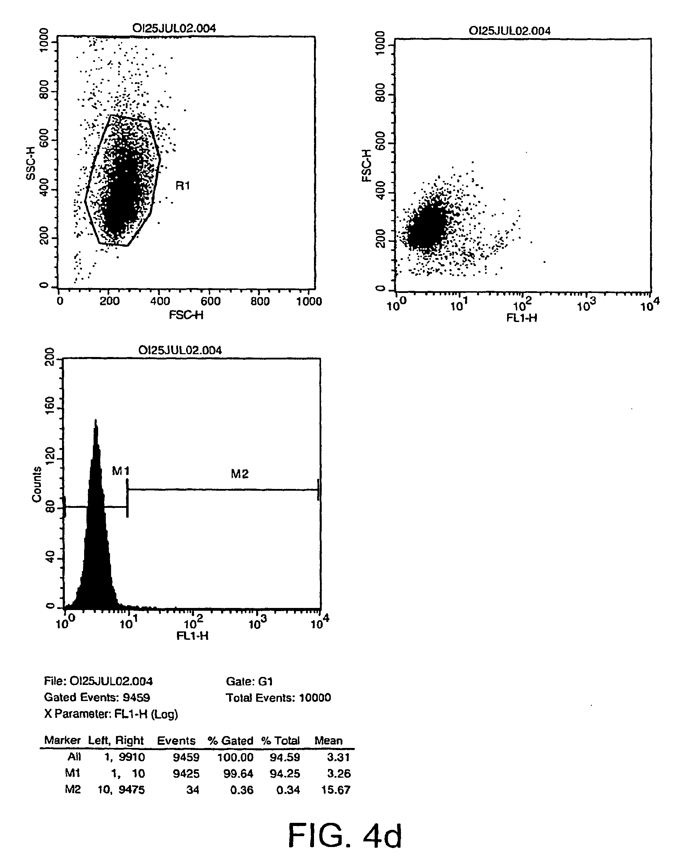

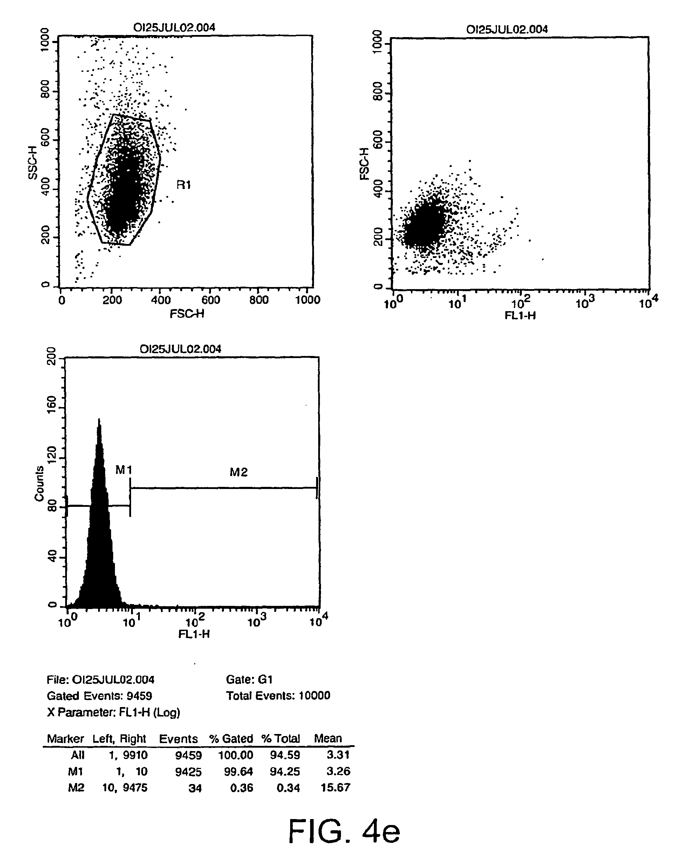

**Figure 4** shows that E5-Fc fusion protein is able to bind to the cell line expressing human Fc receptors. Purified E5-Fc protein was labelled with fluorescein at 3.3 / 1 ratio of Fluo/Protein. The labelled protein (491 µg/ml concentration) was then used for FACS analysis. Human monocyte-like U937 cells which express two types of human FcRs (CD 64 and CD32) were used to assess the ability of E5-Fc fusion protein to bind these receptors. FACS results indicate that E5-Fc fusion protein binds to the U937 cell line (5x10⁵ U-937 cells were incubated with 80ml of the 1:50 dilution of the labelled protein and examined live).
   a. **Fig 4a****:** U-937 cells (control)
   b. **Fig 4b**:U-937 cells incubated with anti CD64 antibody (positive control)
   c. **Fig 4c**:U-937 cells incubated with anti CD32 antibody (positive control)
   d. **Fig 4d**:U-937 cells incubated with anti CD16 antibody (negative control)
   e. **Fig 4e**:U-937 cells incubated with E5-Fc fusion protein
**Figure 5****:** Raj 1 cells (expressing only CD32 receptor) were used for FACS analysis. FACS results demonstrate that E5-Fc chain binds to Raj 1 cells.
   1. Raj 1 cells (control)
   2. Raj 1 cells incubated with anti CD64 antibody (negative control)
   3. Raj 1 cells incubated with anti CD32 antibody (positive control)
   4. Raj 1 cells incubated with anti CD16 antibody (negative control)
   5. Raj 1 cells incubated with E5-Fc fusion protein
**Figure 6** shows the results of Pharmacokinetic Analysis. The figure shows the serum levels in mice following 50µg bolus IV doses of HEL-4 or E5-Fc according to the invention.
**Figure 7** shows the effect of twice weekly injections of TAR1-5-19 on the arthritic scores of the Tg197 mice.
**Figure 8** shows histopathological scoring of the ankle joints from the different treatment groups.
**Figure 9** shows the effect of twice weekly injections of TAR1-5-19 on the group average weights of Tg197 mice.
**Figure 10**: Nucleotide sequence of the alpha factor dAb Fc fusion protein from the start of the alpha factor leader sequence to the EcoRI cloning site.
**Figure 11**: Amino acid sequence of the alpha factor dAb Fc fusion protein, as encoded by the sequence shown in figure 10.

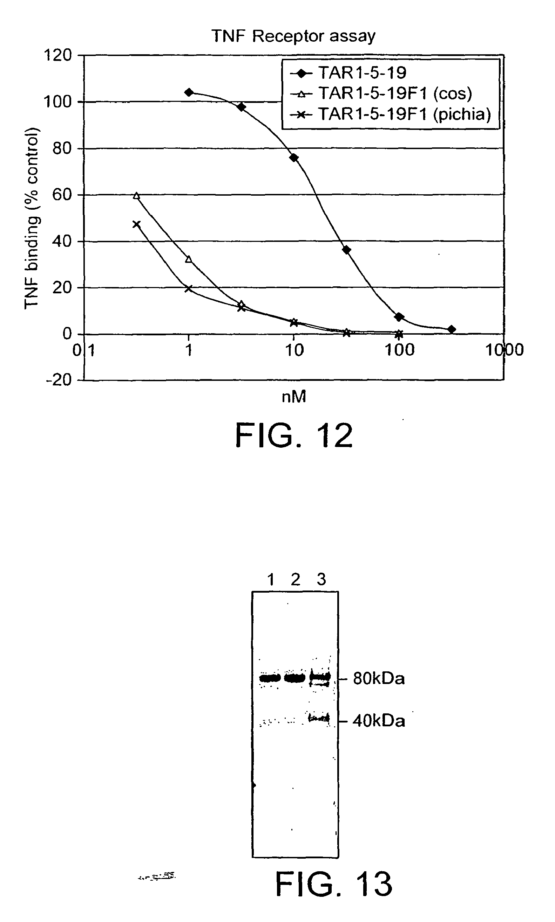

**Figure 12**: Antigen binding activity: Antigen binding activity was determined using a TNF receptor binding assay. A 96 well Nunc Maxisorp plate is coated with a mouse anti-human Fc antibody, blocked with 1% BSA, then TNF receptor 1-Fc fusion is added. The dAb-Fc fusion protein at various concentrations is mixed with 10ng/ml TNF protein and incubated at room temperature for > 1hour. This mixture is added to the TNF receptor 1-Fc fusion protein coated plates, and incubated for 1hour at room temperature. The plates are then washed to remove unbound free dAb-Fc fusion, TNF and dAb-Fc/TNF complexes. The plate was then incubated sequentially with a biotinylated anti-TNF antibody and streptavidin-horse radish peroxidase. The plate was then incubated with the chromogenic horse radish peroxidase substrate TMB. The colour development was stopped with the addition of 1M hydrochloric acid, and absorbance read at 450nm. The absorbance read is proportional to the amount of TNF bound, hence, the TAR1-5-19Fc fusion protein will compete with the TNF receptor for binding of the TNF, and reduce the signal in the assay. The P. pastoris produced protein had an equivalent activity to the mammalian protein in the vitro TNF receptor assay described above.
**Figure 13** shows a 15% non-reducing SDS-PAGE gel showing comparison between TAR1-5-19 Fc fusion protein produced in mammalian cells (lanes 1 and 2) and P. pastoris (lane 3), purified by Protein A affinity. It can be seen that the major bands present in all three lanes are the disulphide linked homodimer at ∼80kDa, and the non-disulphide linked monomer unit at ∼40kDa. Gel filtration on both mammalian and P. pastoris produced protein indicated that under non-SDS-PAGE conditions both species run as homodimers. The minor band below the 80kDa marker represents free Fc protein, without dAb attached, produced through proteolytic attack of the polypeptide linking the dAb and Fc domains.

### Detailed Description of the invention

### Definitions

**Immunoglobulin** This refers to a family of polypeptides which retain the immunoglobulin fold characteristic of antibody molecules, which contains two β sheets and, usually, a conserved disulphide bond. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signalling (for example, receptor molecules, such as the PDGF receptor).

**Domain** A domain is a folded protein structure which retains its tertiary structure independently of the rest of the protein. Generally, domains are responsible for discrete functional properties of proteins, and in many cases may be added, removed or transferred to other proteins without loss of function. By single antibody variable domain we mean a folded polypeptide domain comprising sequences characteristic of antibody variable domains. It therefore includes antibody variable domains, for example in which one or more loops have been replaced by sequences which are not characteristic of antibody variable domains, or antibody variable domains which have been truncated or comprise N- or C-terminal extensions.

**Repertoire** A collection of diverse variants, for example polypeptide variants which differ in their primary sequence. A library used in the present invention will encompass a repertoire of polypeptides comprising at least 1000 members.

**Library** The term library refers to a mixture of heterogeneous polypeptides or nucleic acids. The library is composed of members, which have a single polypeptide or nucleic acid sequence. To this extent, *library* is synonymous with *repertoire*. Sequence differences between library members are responsible for the diversity present in the library. The library may take the form of a simple mixture of polypeptides or nucleic acids, or may be in the form organisms or cells, for example bacteria, viruses, animal or plant cells and the like, transformed with a library of nucleic acids. Preferably, each individual organism or cell contains only one or a limited number of library members. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

A **single-domain-effector group** (dAb-effector group) as herein defined describes an engineered synthetic structure comprising a single variable domain capable of specifically binding one or more epitopes, attached to one or more constant region domains and/or hinge (collectively termed an "effector group"). The variable domain is a light chain domain (V_{L}). In one embodiment, an effector group as herein described comprises an Fc region of an antibody. dAb-effector groups may be combined to form multivalent structures, thus improving the avidity of antigen interaction. For the avoidance of doubt, dAb-effector group immunoglobulin molecules according to the invention are single chain molecules, they are not dual-chain antibodies (for example those described in EP 0 656 946A1). In addition, the term 'dAb-effector group does not include within its scope the naturally occurring dual chain antibodies generated within Camelids nor the four chain structure of IgG molecules. 'dAb-effector groups' according to the present have a half-life which is of sufficient length such that it can produce an *in vivo* biological effect. The present inventors have found that it is the size and nature of the effector group which determines the effector functions of the dAb-effector group as herein defined as well as the *in vivo* half-life of the molecule.

**Antibody** An antibody (for example IgG1, 2, 3 and 4; IgM; IgA; IgD; or IgE) or fragment (such as a Fab, **Dab**, F(ab')₂, Fv, disulphide linked Fv, scFv, diabody) whether derived from any species naturally producing an antibody, or created by recombinant DNA technology; whether isolated from serum, B-cells, hybridomas, transfectomas, yeast or bacteria).

**TAR1-5-19** is a Dab which specifically binds to the target human TNF alpha (TAR1).

**Antigen** A ligand that is bound by a dAb-effector group according to the present invention. Advantageously, single domains may be selected according to their antigen-binding specificity for use in the present invention. The antigen may be a polypeptide, protein, nucleic acid or other molecule. In the case of antibodies and fragments thereof, the antibody binding site defined by the variable loops (L1, L2, L3 and H1, H2, H3) is capable of binding to the antigen.

An **epitope** as referred to herein is a unit of structure conventionally bound by one or more immunoglobulin variable domains, for example an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation of another variable domain.

The term selecting means choosing from a number of different alternatives. Those skilled in that art will be aware of methods of selecting one or more antibody single variable domains. Advantageously, the method involves selecting from a library. Advantageously, the library is a phage display library.

**Universal framework** A single antibody framework sequence corresponding to the regions of an antibody conserved in sequence as defined by Kabat ("Sequences of Proteins of Immunological Interest", US Department of Health and Human Services) or structure as defined by Chothia and Lesk, (1987) J. Mol. Biol. 196:910-917. The invention provides for the use of a single framework, or a set of such frameworks, which has been found to permit the derivation of virtually any binding specificity though variation in the hypervariable regions alone.

**Specific generic ligand** A ligand that binds to all members of a repertoire. Generally, not bound through the antigen binding site. Examples include protein A and protein L.

As used herein, the term "**human origin**" means that at some point in the derivation of a sequence in question, a human sequence was used as a source of nucleic acid sequence. An analogous meaning applies to the term **"Camelid origin."**

As used herein, the phrase "**increased half-life**" means that a given dAb-effector group has at least a 25% longer serum half-life relative to the same dAb without the effector. Increased half-lives are preferably at least 30% longer, 40% longer, 50% longer, 75% longer, 100% longer, 3X longer, 5X longer, 10X longer, 20X longer, 50X longer or more.

As used herein, the term "**selecting**" is to be understood to require the application of a technique or selective pressure, thus permitting the isolation of one or more items from among a population based on one or more characteristics possessed by the selected item(s) that is/are not possessed by the other members of the population.

### Detailed description of the invention

### General Techniques

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. which are incorporated herein by reference) and chemical methods.

### Preparation of dAb-effector groups according to the present invention

dAb-effector groups may be prepared according to previously established techniques, used in the field of antibody engineering, for the preparation of scFv, "phage" antibodies and other engineered antibody molecules. Techniques for the preparation of antibodies, and in particular bispecific antibodies, are for example described in the following reviews and the references cited therein: Winter & Milstein, (1991) Nature 349:293-299; Plueckthun (1992) Immunological Reviews 130:151-188; Wright et al., (1992) Crti. Rev. Immunol.12:125-165; Holliger, P. & Winter, G. (1993) Curr. Op. Biotechn. 4, 446-449; Carter, et al. (1995) J. Hematother. 4, 463-470; Chester, K.A. & Hawkins, R.E. (1995) Trends Biotechn. 13, 294-300; Hoogenboom, H.R. (1997) Nature Biotechnol. 15, 125-126; Fearon, D. (1997) Nature Biotechnol. 15, 618-619; Plückthun, A. & Pack, P. (1997) Immunotechnology 3, 83-105; Carter, P. & Merchant, A.M. (1997) Curr. Opin. Biotechnol. 8, 449-454; Holliger, P. & Winter, G. (1997) Cancer Immunol. Immunother. 45,128-130.

The techniques employed for selection of the variable domains employ libraries and selection procedures which are known in the art. Natural libraries (Marks et al. (1991) J. Mol. Biol., 222: 581; Vaughan et al. (1996) Nature Biotech., 14: 309) which use rearranged V genes harvested from human B cells are well known to those skilled in the art. Synthetic libraries (Hoogenboom & Winter (1992) J. Moli. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97) are prepared by cloning immunoglobulin V genes, usually using PCR. Errors in the PCR process can lead to a high degree of randomisation. V_{H} and/or V_{L} libraries may be selected against target antigens or epitopes separately, in which case single domain binding is directly selected for, or together.

A preferred method for synthesising a 'dAb-effector group' according to the present invention comprises using a selection system in which a repertoire of variable domains is selected for binding to an antigen or epitope. The single domains selected are then attached to an effector group.

Suitable effector groups include any of those selected from the group consisting of the following: an effector group comprising at least an antibody light chain constant region (CL), an antibody CH1 heavy chain domain, an antibody CH2 heavy chain domain, an antibody CH3 heavy chain domain, or any combination thereof. In addition to the one or more constant region domains, an effector group may also comprise a hinge region of an antibody (such a region normally being found between the CH1 and CH2 domains of an IgG molecule). According to an alternative embodiment of the invention, the 'dAb-effector group' is a single variable domain attached to the hinge region derived from an antibody molecule.

In an alternative embodiment of this aspect of the invention, the effector group is based on a Fab antibody fragment. That is, it comprises an antibody fragment comprising a V_{L} domain and optionally a V_{H} domain attached to one or more constant region domains making up a Fab fragment. One skilled in the art will appreciate that such a fragment comprises only one variable domain. Such Fab effector groups are illustrated in Fig lg. In the 2 chain embodiment shown in Fig lg (ie, the 2-chain embodiment), the single variable domains each forms a respective epitope or antigen binding site. Thus, the single variable domains do not together form a single binding site. The epitope or antigen specificity of the variable domains is different.

In a further preferred embodiment of this aspect of the invention, an effector group according to the present invention is an Fc region of an IgG molecule.

### A. Library vector systems

A variety of selection systems are known in the art which are suitable for use in the present invention. Examples of such systems are described below.

Bacteriophage lambda expression systems may be screened directly as bacteriophage plaques or as colonies of lysogens, both as previously described (Huse et al. (1989) Science, 246: 1275; Caton and Koprowski- (1990) Proc. Natl. Acad. Sci. U. S. A., 87**;** Mullinax et al. (1990) Proc. Natl. Acad. Sci. U. S. A., 87: 8095; Persson et al. (1991) Proc. Natl. Acad Sci. U.S.A., 88: 2432) and are of use in the invention. Whilst such expression systems can be used to screening up to 10⁶ different members of a library, they are not really suited to screening of larger numbers (greater than 10⁶ members).

Of particular use in the construction of libraries are selection display systems, which enable a nucleic acid to be linked to the polypeptide it expresses. As used herein, a selection display system is a system that permits the selection, by suitable display means, of the individual members of the library by binding the generic and/or target ligands.

Selection protocols for isolating desired members of large libraries are known in the art, as typified by phage display techniques. Such systems, in which diverse peptide sequences are displayed on the surface of filamentous bacteriophage (Scott and Smith (1990) Science, 249: 386), have proven useful for creating libraries of antibody fragments (and the nucleotide sequences encoding them) for the *in vitro* selection and amplification of specific antibody fragments that bind a target antigen (McCafferty et al., WO 92/01047). The nucleotide sequences encoding the V_{H} and V_{L} regions are linked to gene fragments which encode leader signals that direct them to the periplasmic space of *E*. *coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty et al. (1990) Nature, 348: 552; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al*. (1991) supra; Barbas *et al*. (1992) supra; Hawkins and Winter (1992) J. Immunol., 22: 867; Marks et al., 1992, J. Biol. Chem., 267: 16007; Lerner et al. (1992) Science, 258: 1313, incorporated herein by reference).

One particularly advantageous approach has been the use of scFv phage-libraries (Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al*. (1990) supra; Clackson et al. (1991) Nature, 352: 624; Marks et al. (1991) J. Mol. Biol., 222: 581; Chiswell et al. (1992) Trends Biotech., 10: 80; Marks et al. (1992) J. Biol. Chem., 267). Various embodiments of scFv libraries displayed on bacteriophage coat proteins have been described. Refinements of phage display approaches are also known, for example as described in WO96/06213 and WO92/01047 (Medical Research Council *et al*.) and WO97/08320 (Morphosys), which are incorporated herein by reference.

Other systems for generating libraries of polypeptides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bach (1990) Nucleic Acids Res., 18: 3203; Beaudry and Joyce (1992) Science, 257: 635; WO92/05258 and WO92/14843). In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection.

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

### B. Library Construction.

Libraries intended for use in selection may be constructed using techniques known in the art, for example as set forth above, or may be purchased from commercial sources. Libraries which are useful in the present invention are described, for example, in WO99/20749. Once a vector system is chosen and one or more nucleic acid sequences encoding polypeptides of interest are cloned into the library vector, one may generate diversity within the cloned molecules by undertaking mutagenesis prior to expression; alternatively, the encoded proteins may be expressed and selected, as described above, before mutagenesis and additional rounds of selection are performed. Mutagenesis of nucleic acid sequences encoding structurally optimised polypeptides is carried out by standard molecular methods. Of particular use is the polymerase chain reaction, or PCR, (Mullis and Faloona (1987) Methods Enzymol., 155: 335, herein incorporated by reference). PCR, which uses multiple cycles of DNA replication catalysed by a thermostable, DNA-dependent DNA polymerase to amplify the target sequence of interest, is well known in the art. The construction of various antibody libraries has been discussed in Winter et al. (1994) Ann. Rev. Immunology 12, 433-55, and references cited therein.

PCR is performed using template DNA (at least 1fg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionized water to a total volume of 25 µl. Mineral oil is overlaid and the PCR is performed using a programmable thermal cycler. The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenized, mismatch is required, at least in the first round of synthesis. The ability to optimise the stringency of primer annealing conditions is well within the knowledge of one of moderate skill in the art. An annealing temperature of between 30 °C and 72 °C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1-5 minutes, depending on the length of the amplified product). Final extension is generally for 4 minutes at 72°C, and may be followed by an indefinite (0-24 hour) step at 4°C.

### C. Attaching single variable domains to effector groups according to the present invention

Domains according to the invention, once selected, may be attached to effector groups as herein described by a variety of methods known in the art, including covalent and noncovalent methods.

Preferred methods include the use of polypeptide linkers, as described, for example, in connection with scFv molecules (Bird et al., (1988) Science 242:423-426). Linkers may be flexible, allowing the two single domains to interact. The linkers used in diabodies, which are less flexible, may also be employed (Holliger et al., (1993) PNAS (USA) 90:6444-6448).

Variable domains may be attached to effector groups using methods other than linkers. For example, the use of disulphide bridges, provided through naturally-occurring or engineered cysteine residues, may be exploited.

Other techniques for attaching variable domains of immunoglobulins to effector groups of the present invention may be employed as appropriate.

The length and nature of the linker may affect the physical characteristics of the dAb effector molecule. For example, the linkers may facilitate the association of the domains, such as by incorporation of small amino acid residues in opportune locations. Alternatively, a suitable rigid structure may be designed which will keep the effector group and the variable domain in close physical proximity to one another.

### D 'dAb-effector groups' according to the present invention.

According to the present invention, single V_{L} variable domains are attached to an effector group via means herein described.

### (a) Preparation of dAb-effector group of the present invention

A dAb-effector group according to the present invention may be derived from any species naturally producing an antibody, or created by recombinant DNA technology; whether isolated from serum, B-cells, hybridomas, transfectomas, yeast or bacteria.

The single variable domain and the effector group according to the present invention may be on the same polypeptide chain. Alternatively, they may be on separate polypeptide chains. In the case that they are on the same polypeptide chain they may be linked by a linker. Preferably, the linker is a peptide sequence, as described above.

The single variable domain and the effector group may be covalently or non-covalently associated. In the case that they are covalently associated, the covalent bonds may be disulphide bonds.

In the case that the variable domains are selected from V-gene repertoires selected for instance using phage display technology as herein described, then these variable domains comprise a universal framework region, such that is they may be recognised by a specific generic ligand as herein defined. The use of universal frameworks, generic ligands and the like is described in WO99/20749. Examples of preferred germ-line gene segments for preparation of dAB-effector groups according to the invention include any of those selected from the group consisting of the following: DP38, DP45, DP47 and DPK9.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary epitope.

In a further aspect, the present invention provides nucleic acid encoding at least a single domain-effector group antibody as herein defined.

The variable regions may be derived from antibodies directed against target antigens or epitopes. Alternatively they may be derived from a repertoire of single antibody domains such as those expressed on the surface of filamentous bacteriophage. Selection may be performed as described below.

In general, the nucleic acid molecules and vector constructs required for the performance of the present invention may be constructed and manipulated as set forth in standard laboratory manuals, such as Sambrook et al. (1989) Molecular Cloning: A Laboratory Manual, Cold Spring Harbor, USA.

The manipulation of nucleic acids in the present invention is typically carried out in recombinant vectors.

Thus in a further aspect, the present invention provides a vector comprising nucleic acid encoding at least a single domain-effector group as herein defined.

As used herein, vector refers to a discrete element that is used to introduce heterologous DNA into cells for the expression and/or replication thereof. Methods by which to select or construct and, subsequently, use such vectors are well known to one of ordinary skill in the art. Numerous vectors are publicly available, including bacterial plasmids, bacteriophage, artificial chromosomes and episomal vectors. Such vectors may be used for simple cloning and mutagenesis; alternatively gene expression vector is employed. A vector of use according to the invention may be selected to accommodate a polypeptide coding sequence of a desired size, typically from 0.25 kilobase (kb) to 40 kb or more in length. A suitable host cell is transformed with the vector after *in vitro* cloning manipulations. Each vector contains various functional components, which generally include a cloning (or "polylinker") site, an origin of replication and at least one selectable marker gene. If given vector is an expression vector, it additionally possesses one or more of the following: enhancer element, promoter, transcription termination and signal sequences, each positioned in the vicinity of the cloning site, such that they are operatively linked to the gene encoding a polypeptide repertoire member according to the invention.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available in the growth media.

Since the replication of vectors according to the present invention is most conveniently performed in *E. coli*, an E. coli-selectable marker, for example, the β-lactamase gene that confers resistance to the antibiotic ampicillin, is of use. These can be obtained from E. coli plasmids, such as pBR322 or a pUC plasmid such as pUC18 or puck19 or pUC119.

Expression vectors usually contain a promoter that is recognised by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Delgarno sequence operably linked to the coding sequence.

The preferred vectors are expression vectors that enable the expression of a nucleotide sequence corresponding to a polypeptide library member. Thus, selection with the first and/or second antigen or epitope can be performed by separate propagation and expression of a single clone expressing the polypeptide library member or by use of any selection display system. As described above, the preferred selection display system is bacteriophage display. Thus, phage or phagemid vectors may be used. The preferred vectors are phagemid vectors which have an *E. coli.* origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector contains a β-lactamase gene to confer selectivity on the phagemid and a 1ac promoter upstream of a expression cassette that consists (N to C terminal) of a pelB leader sequence (which directs the expressed polypeptide to the periplasmic space), a multiple cloning site (for cloning the nucleotide version of the library member), optionally, one or more peptide tag (for detection), optionally, one or more TAG stop codon and the phage protein pIII. Thus, using various suppressor and non-suppressor strains of *E. coli* and with the addition of glucose, iso-propyl thio-β-D-galactoside (IPTG) or a helper phage, such as VCS M 13, the vector is able to replicate as a plasmid with no expression, produce large quantities of the polypeptide library member only or produce phage, some of which contain at least one copy of the polypeptide-pIII fusion on their surface.

Construction of vectors according to the invention employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, *in situ* hybridisation, immunocytochemistry or sequence analysis of nucleic acid or protein molecules. Those skilled in the art will readily envisage how these methods may be modified, if desired.

### (b) Structure of dAb-effector groups according to the invention

A single-domain antibody-effector group (dAb-effector group) as herein defined describes an engineered antibody molecule comprising a single variable domain capable of specifically binding one or more epitopes, attached to one or more constant region domains (effector groups). The variable domain is a light chain domain (V_{L}).

Suitable effector groups include any of those selected from the group consisting of the following: an effector group comprising at least an antibody light chain constant region (CL), an antibody CH1 heavy chain domain, an antibody CH2 heavy chain domain, an antibody CH3 heavy chain domain, or any combination thereof. In addition to the one or more constant region domains, an effector group may also comprise a hinge region of an antibody (such a region normally being found between the CH1 and CH2 domains of an IgG molecule). Advantageously, an effector group as herein described comprises an Fc region of an antibody. More advantageously a dAb-effector group according to the present invention is a V_{L}-Fc.

In an alternative embodiment of this aspect of the invention, the effector group is based on a Fab antibody fragment. That is, it comprises an antibody fragment comprising a V_{L} domain attached to the constant region domains making up a Fab fragment. One skilled in the art will appreciate that such a fragment comprises only one variable domain.

In one embodiment, a dAb-effector group according to the invention has a tβ half-life of any of those tβ half-lifes selected from the group consisting of the following: 12 hours or more, 24 hours or more, 2 days or more, 3 days or more, 4 days or more, 5 days or more, 6 days or more, 7 days or more, 8 days or more, 9 days or more, 10 days or more, 11 days or more, 12 days or more, 13 days or more, 14 days or more, 15 days or more or 20 days or more. Advantageously a dAb-effector group or composition according to the invention will have a tβ half life in the range 12 to 60 hours. In a further embodiment, it will have a t half-life of a day or more. In a further embodiment still, it will be in the range 12 to 26 hours.

Advantageously, a dAb-effector group according to the present invention comprises or consists of an V_{L}-Fc having a tβ half-life of a day or more, two days or more, 3 days or more, 4 days or more, 5 days or more, 6 days or more or 7 days or more. Most advantageously, a dAb-effector group according to the present invention comprises or consists of an V_{L}-Fc having a tβ half-life of a day or more.

According to the present invention, advantageously, a dAb-effector group according comprises an effector group consisting of the constant region domains CH2 and/or CH3, preferably CH2 and CH3, either with or without a hinge region as described herein, wherein the dAb-effector group has a tβ half-life of a day or more, two days or more, 3 days or more, 4 days or more, 5 days or more, 6 days or more or 7 days or more. More advantageously, a dAb-effector group according to the present invention comprises an effector group consisting of the constant region domains CH2 and/or CH3 wherein the dAb-effector group has a tβ half-life of a day or more.

### Immunoglobulin scaffolds

Each single variable domain comprises an immunoglobulin scaffold and one or more CDRs which are involved in the specific interaction of the domain with one or more epitopes.

### i. Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol., 196: 901; Chothia et al. (1989) Nature, 342: 877). Analysis of loop lengths and key residues has therefore enabled prediction of the main-chain conformations of H1, H2, L1, L2 and L3 found in the majority of human antibodies (Chothia et al. (1992) J. Mol. Biol., 227: 799; Tomlinson et al. (1995) EMBO J., 14: 4628; Williams et al. (1996) J. Mol. Biol., 264: 220). Although the H3 region, is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1).

The dAb-effector groups of the present invention are advantageously assembled from libraries of domains, such as libraries of V_{L} domains. For use in the present invention, libraries of antibody polypeptides are designed in which certain loop lengths and key residues have been chosen to ensure that the main-chain conformation of the members is known. Advantageously, these are real conformations of immunoglobulin superfamily molecules found in nature, to minimise the chances that they are non-functional. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use in the invention to assess the number of different main-chain conformations encoded by antibodies, to predict the main-chain conformation based on antibody sequences and to chose residues for diversification which do not affect the canonical structure. It is known that, in the human V_{κ} domain, the L1 loop can adopt one of four canonical structures, the L2 loop has a single canonical structure and that 90% of human V_{κ} domains adopt one of four or five canonical structures for the L3 loop (Tomlinson *et al.* (1995) supra); thus, in the V_{κ} domain alone, different canonical structures can combine to create a range of different main-chain conformations. Given that the V_{λ} domain encodes a different range of canonical structures for the L1, L2 and L3 loops and that V_{κ} and V_{.λ}domains can pair with any V_{H} domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a preferred aspect, the dAb-effector groups of the invention possess a single known main-chain conformation.

The single main-chain conformation that is chosen is preferably commonplace among molecules of the immunoglobulin superfamily type in question. A conformation is commonplace when a significant number of naturally occurring molecules are observed to adopt it. Accordingly, in a preferred aspect of the invention, the natural occurrence of the different main-chain conformations for each binding loop of an immunoglobulin superfamily molecule are considered separately and then a naturally occurring immunoglobulin superfamily molecule is chosen which possesses the desired combination of main-chain conformations for the different loops. If none is available, the nearest equivalent may be chosen. It is preferable that the desired combination of main-chain conformations for the different loops is created by selecting germline gene segments which encode the desired main-chain conformations. It is more preferable, that the selected germline gene segments are frequently expressed in nature, and most preferable that they are the most frequently expressed of all natural germline gene segments.

In designing single variable domains or libraries thereof the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of V_{H}(39%), L2 - CS 1 (100%), L3 - CS 1 of V_{H}(36%) (calculation assumes a _{κ}:λ ratio of 70:30, Hood et al. (1967) Cold Spring Harbor Symp. Quant. Biol., 48: 133). For H3 loops that have canonical structures, a CDR3 length (Kabat *et al.* (1991) *Sequences of proteins of immunological interest*, U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the V_{H} segment 3-23 (DP-47), the J_{H} segment JH4b, the V_{κ} segment O2/O12 (DPK9) and the J_{κ} segment J_{κ}1. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five or for all six of the antigen binding loops can be determined. Here, it is preferable that the chosen conformation is commonplace in naturally occurring antibodies and most preferable that it is observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

### b. Diversification of the canonical sequence

The desired diversity is typically generated by varying the selected molecule at one or more positions. The positions to be changed can be chosen at random or are preferably selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins et al. (1992) J. Mol. Biol., 226: 889), chemical mutagenesis (Deng et al. (1994) J Biol. Chem., 269: 9533) or bacterial mutator strains (Low et al. (1996) J Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas et al. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97). Such diversification has been extended to include some or all of the other antigen binding loops (Crameri et al. (1996) Nature Med., 2: 100; Riechmann et al. (1995) Bio/Technology, 13: 475; Morphosys, WO97/08320, supra).

Since loop randomisation has the potential to create approximately more than 10¹⁵ structures for H3 alone and a similarly large number of variants for the other five loops, it is not feasible using current transformation technology or even by using cell free systems to produce a library representing all possible combinations. For example, in one of the largest libraries constructed to date, 6 x 10¹⁰ different antibodies, which is only a fraction of the potential diversity for a library of this design, were generated (Griffiths *et al.* (1994) supra).

In addition to the removal of non-functional members and the use of a single known main-chain conformation, these limitations are addressed by diversifying only those residues which are directly involved in creating or modifying the desired function of the molecule. For many molecules, the function will be to bind a target and therefore diversity should be concentrated in the target binding site, while avoiding changing residues which are crucial to the overall packing of the molecule or to maintaining the chosen main-chain conformation.

### E Characterisation of dAb-effector groups according to the present invention .

The binding of single domain antibody-effector groups (dAb-effector group) according to the invention to its specific antigens or epitopes can be tested by methods which will be familiar to those skilled in the art and include ELISA. In a preferred embodiment binding is tested using monoclonal phage ELISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12, 433-55 and references cited therein).

The diversity of the selected phage monoclonal antibodies may also be assessed by gel electrophoresis of PCR products (Marks *et al.* 1991, *supra*; Nissim *et al.* 1994 *supra*), probing (Tomlinson et al., 1992) J. Mol. Biol. 227, 776) or by sequencing of the vector DNA.

### F. Nucleic acid constructs according to the present invention.

In general, the nucleic acid molecules and vector constructs required for the performance of the present invention may be constructed and manipulated as set forth in standard laboratory manuals, such as Sambrook et al. (1989) Molecular Cloning: A Laboratory Manual, Cold Spring Harbor, USA.

The manipulation of nucleic acids in the present invention is typically carried out in recombinant vectors.

As used herein, vector refers to a discrete element that is used to introduce heterologous DNA into cells for the expression and/or replication thereof. Methods by which to select or construct and, subsequently, use such vectors are well known to one of moderate skill in the art. Numerous vectors are publicly available, including bacterial plasmids, bacteriophage, artificial chromosomes and episomal vectors. Such vectors may be used for simple cloning and mutagenesis; alternatively gene expression vector is employed. A vector of use according to the invention may be selected to accommodate a polypeptide coding sequence of a desired size, typically from 0.25 kilobase (kb) to 40 kb or more in length. A suitable host cell is transformed with the vector after *in vitro* cloning manipulations. Each vector contains various functional components, which generally include a cloning (or "polylinker") site, an origin of replication and at least one selectable marker gene. If a given vector is an expression vector, it additionally possesses one or more of the following: enhancer element, promoter, transcription termination and signal sequences, each positioned in the vicinity of the cloning site, such that they are operatively linked to the gene encoding a polypeptide.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available in the growth media.

Since the replication of vectors is most conveniently performed in *E. coli,* an *E. coli-*selectable marker, for example, the β-lactamase gene that confers resistance to the antibiotic ampicillin, is of use. These can be obtained from *E. coli* plasmids, such as pBR322 or a pUC plasmid such as pUC 18 or pUC 19 or pUC119.

Expression vectors usually contain a promoter that is recognised by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Delgamo sequence operably linked to the coding sequence.

The preferred vectors are expression vectors that enables the expression of a nucleotide sequence corresponding to a polypeptide. Thus, selection with antigen can be performed by separate propagation and expression of a single clone expressing the polypeptide or by use of any selection display system. As described above, the preferred selection display system is bacteriophage display. Thus, phage or phagemid vectors may be used. The preferred vectors are phagemid vectors which have an *E. coli* origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector contains a β-lactamase gene to confer selectivity on the phagemid and a lac promoter upstream of a expression cassette that consists (N to C terminal) of a pelB leader sequence (which directs the expressed polypeptide to the periplasmic space), a multiple cloning site (for cloning the nucleotide version of the polypeptide), optionally, one or more peptide tag (for detection), optionally, one or more TAG stop codon and the phage protein pIII. Thus, using various suppressor and non-suppressor strains of *E. coli* and with the addition of glucose, iso-propyl thio-β-D-galactoside (IPTG) or a helper phage, such as VCS M13, the vector is able to replicate as a plasmid with no expression, produce large quantities of the polypeptide only or produce phage, some of which contain at least one copy of the polypeptide-pIII fusion on their surface.

Construction of vectors according to the invention employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, *in situ* hybridisation, immunocytochemistry or sequence analysis of nucleic acid or protein molecules. Those skilled in the art will readily envisage how these methods may be modified, if desired.

### G: Use of dAb-effector groups according to the invention

dAb-effector groups selected according to the method of the present invention may be employed in *in vivo* therapeutic and prophylactic applications, *in vitro* and *in vivo* diagnostic applications, *in vitro* assay and reagent applications, and the like. For example the dAb-effector groups may be used in antibody based assay techniques, such as ELISA techniques, according to methods known to those skilled in the art.

Those skilled in the art will appreciate that the dAb-effector groups of the invention can be prepared according to a desired or predetermined antigen binding specificity. In addition, the method of the invention permits the synthesis of dAb-effector groups with a desired effector group. In this way the effector functions can be designed into the dAb-effector group. In addition, the present inventors have found that the presence of the effector group increases the *in vivo* half life of the molecule.

As alluded to above, the dAb-effector groups according to the invention are of use in diagnostic, prophylactic and therapeutic procedures. Single domain-effector group antibodies (dAb-effector groups) selected according to the invention are of use diagnostically in Western analysis and *in situ* protein detection by standard immunohistochemical procedures; for use in these applications, the antibodies of a selected repertoire may be labelled in accordance with techniques known to the art. In addition, such antibody polypeptides may be used preparatively in affinity chromatography procedures, when complexed to a chromatographic support, such as a resin. All such techniques are well known to one of skill in the art.

Substantially dAb-effector groups according to the present invention of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected dAb-effector groups may be used diagnostically and/or therapeutically (including extracorporeally) or in developing and performing assay procedures, immunofluorescent stainings and the like (Lefkovite and Pernis, (1979 and 1981) Immunological Methods, Volumes I and II, Academic Press, NY).

The dAb-effector groups of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the dAb-effector groups in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton, New York, pp. 179-213; McFarlin et al. (1973) Science, 179: 478: and Satoh et al. (1987) J. Immunol., 138: 179).

Generally, the dAb-effector groups will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The dAb-effector groups of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the dAb-effector groups of the present invention, or even combinations of dAb-effector groups according to the present invention having different specificities, such as dAb-effector groups having variable domains selected using different target ligands, whether or not they are pooled prior to administration.

The route of administration of pharmaceutical compositions according to the invention may be any of those commonly known to those of ordinary skill in the art. For therapy, including without limitation immunotherapy, the dAb-effector groups and compositions of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

The dAb-effector groups of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the dAb-effector groups or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of selected antibody, receptor (e.g. a T-cell receptor) or binding protein thereof *per* kilogram of body weight, with doses of 0.05 to 2.0 mg/kg/dose being more commonly used. For prophylactic applications, compositions containing the dAb-effector groups or cocktails thereof may also be administered in similar or slightly lower dosages.

A composition containing a dAb-effector group or cocktail thereof according to the present invention may be utilised in prophylactic and therapeutic settings to aid in the alteration, inactivation, killing or removal of a select target cell population in a mammal. In addition, the dAb-effector groups described herein may be used extracorporeally or *in vitro* selectively to kill, deplete or otherwise effectively remove a target cell population from a heterogeneous collection of cells. Blood from a mammal may be combined extracorporeally with the selected antibodies, cell-surface receptors or binding proteins thereof whereby the undesired cells are killed or otherwise removed from the blood for return to the mammal in accordance with standard techniques.

The invention is further described, for the purposes of illustration only, in the following examples.

### Example 1. Creation of dAb-Fc fusion constructs

This example demonstrates a method for making V*_{κ}*-Fc and V_{H}-Fc fusions (for both fusions, Fc is derived from IgG1, the Fc=hinge-CH2-CH3). A β-galactosidase binding V_{κ} dAb E5 was used to create V_{κ}-Fc fusion and an alkaline phosphatase (APS) binding V_{H} dAb VH2 was used to create V_{H} -Fc fusion (sequences of V_{κ} dAb E5 and V_{H} dAb VH2 are shown in Table 1a).

Hind *III* and Not I restriction sites were introduced onto the 5'and 3'ends, respectively, of the E5 and VH2 dAbs using oligonucleotides VK5Hind, VH5Hind and VH3Not (Table 1a, note that there was no need to introduce Not *I* site onto the 3' end of the E5 dAb, as it already exists).

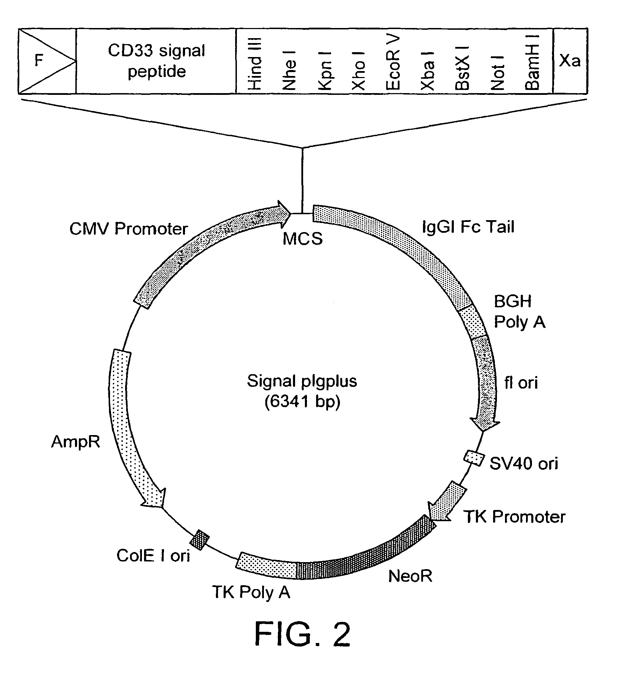

To create E5-Fc and VH2-Fc fusions, Hind *III*/Not *I* digested fragments containing E5 V_{κ} dAb and VH2 V_{H} dAb were then ligated into Hind *III*/Not *I* digested Signal pIgplus vector (R&D Systems Europe Ltd, Figure 2). Ligation mixtures were transformed into competent *E.coli* TG1 cells and recombinant clones were verified by colony PCR screening and sequencing using PIG5SEQ and PIG3SEQ oligonucleotides (Table 1b).

**Table 1a**

| **Primer** | **Sequence (5' to 3')** |
|---|---|
| VK5HTND | CCC AAG CTT GAC ATC CAG ATG ACC CAG TCT CC |
| VH5HIND | CCC AAG CTT GAG GTG CAG CTG TTG GAG TCT GG |
| VH3NOT | TTT TCC TTT TGC GGC CGC GCT CGA GAC GGT GAC CAG GGT TCC |
| PIG5SEQ | ACT CAC TAT AGG GAG ACC CA |
| PIG3SEQ | CAT GTG TGA GGT TTG TCA CAA |

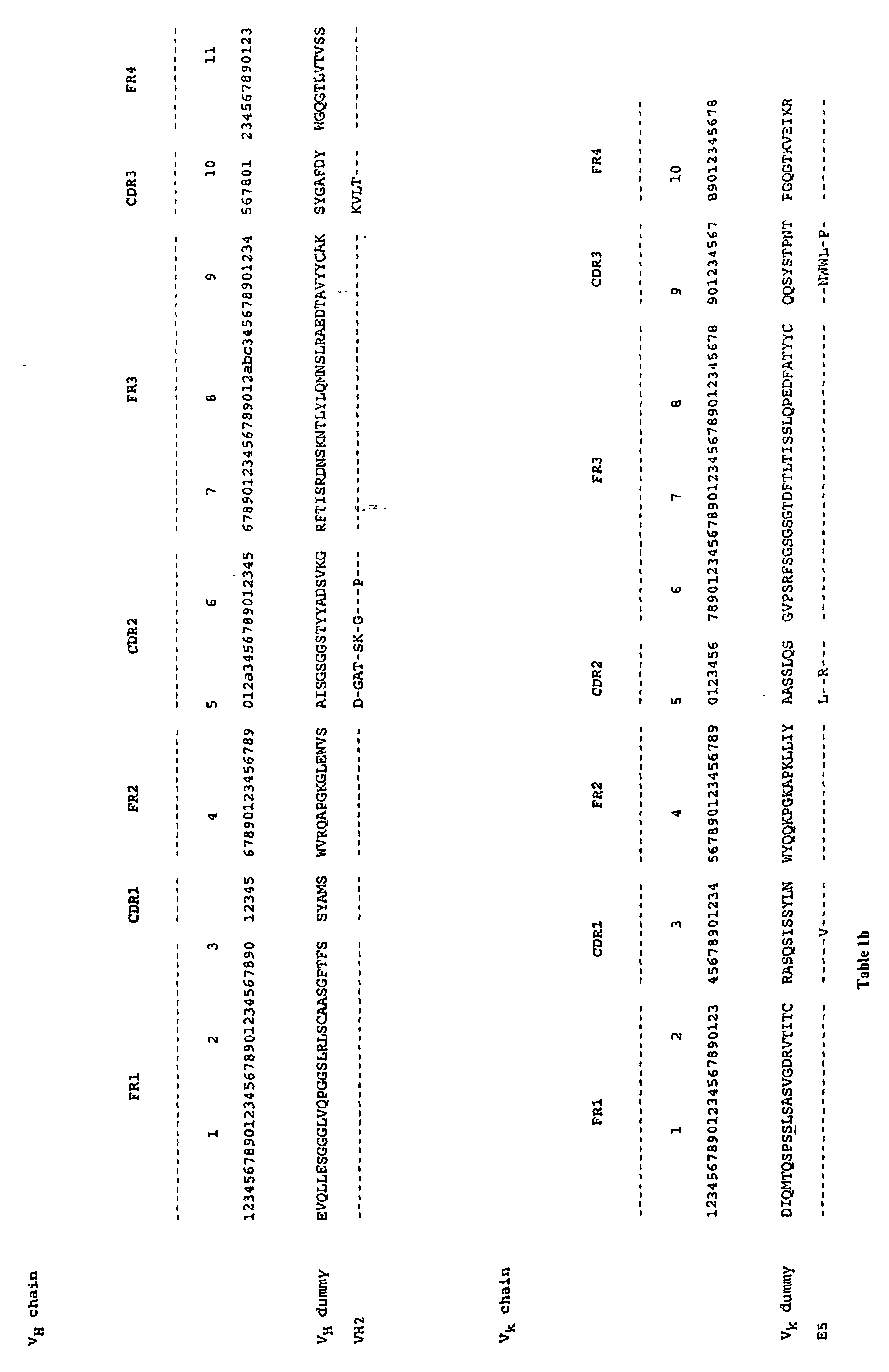

### Example 2. Expression of the dAb-Fc fusion proteins in mammalian cells

This example demonstrates that E5-Fc and VH2-Fc fusions (Example 1) could be expressed in mammalian cells and that the produced proteins retain antigen specificity of the parental dAbs.

Three mammalian cell lines (COS 1, COS7 and CHO) were transfected with E5 dAb in pIgplus and VH2 dAb in pIgplus plasmid DNA (Example 1) using FuGENE 6 Transfection Reagent (Roche). Stably transformed cell lines were generated using selection medium containing G418 (1mg/ml for COS1 and COS7 cells and 0.5mg/ml for CHO cells).

To check the expression of the dAb-Fc fusion proteins, 25ml of the spent tissue culture medium from transfected cells were collected, filtered using 0.45µ filter and then passed through Protein A Sepharose column. dAb-Fc fusions were eluted using 1.6ml 0.1M Glycine pH 2.0 into 0.4ml 1M Tris, pH 9.0. 50 µl of the resulting 2ml sample was tested in ELISA (standard ELISA protocol was followed) 96-well plates were coated with 100 µl of APS and β-galactosidase at 10 µg/ml concentration in PBS overnight at 4C. Detection was performed using anti human IgG (Fc specific) -HRP conjugate (Sigma). ELISA results demonstrate that all cell lines are expressing cLAb-Fc fusions of correct specificities (Figure 3c). No cross-reactivity with irrelevant antigens (APS for E5-Fc and β-galactosidase for VH2-Fc) was observed (Figure 3c).

Analysis of the dAb-Fc chains on the SDS (non-reducing) gel indicates that they are mainly produced as dimers (disulfide bridged at the hinge) with MW of approx. 80kDa (Figure 3a). A 40kDa band is also visible on the gel (Figure 3a), indicating that some of the protein is also present in the monomeric form.

Staining for glycosylation revealed that E5-Fc protein is glycosylated (Figure 3b).

Following optimisation of expression and purification procedures, the yield of E5-Fc fusion protein from COS1 cells is 20mg/l. The expression level of VH2-Fc fusion is lower.

### Example 3. Binding of the E5-Fc fusion protein to the cell line expressing human Fc receptors

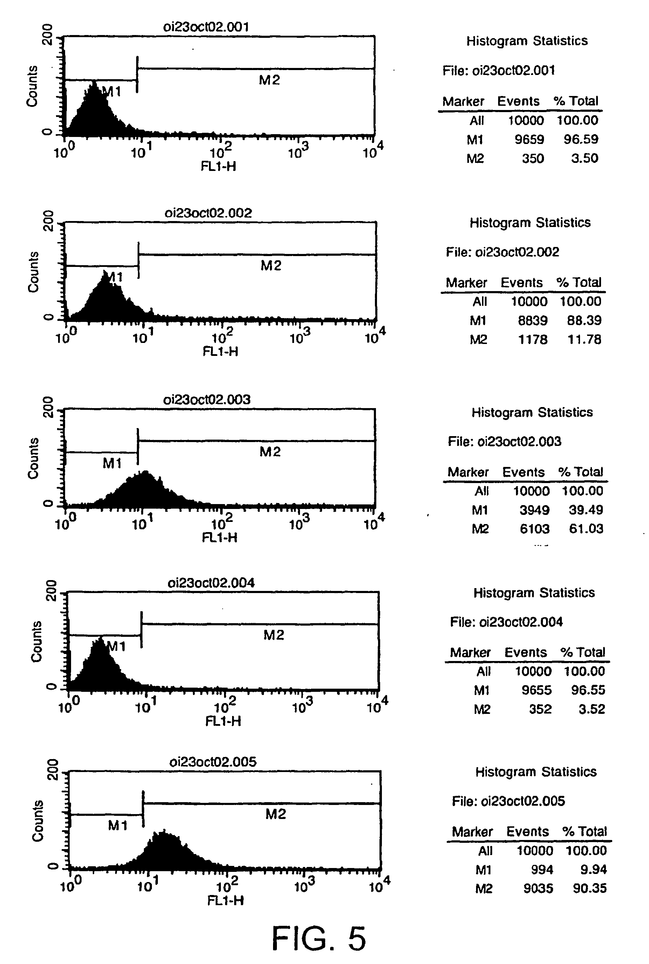

This example demonstrates that E5-Fc fusion protein is able to bind to the cell line expressing human Fc receptors. Purified E5-Fc protein was labelled with fluorescein at 3.3 / 1 ratio of Fluo/Protein. The labelled protein (491 µg/ml concentration) was then used for FACS analysis. Human monocyte-like U937 cells which express two types of human FcRs (CD 64 and CD32) were used to assess the ability of E5-Fc fusion protein to bind these receptors. FACS results indicate that E5-Fc fusion protein binds to the U937 cell line (5x10⁵ U-937 cells were incubated with 80µl of the 1:50 dilution of the labelled protein and examined live) (Figure 4). Receptor blocking studies on U-937 cells indicated that E5-Fc chain binds primarily to CD32 receptor (data not shown). To confirm this result, Raj 1 cells (expressing only CD32 receptor) were used for FACS analysis. FACS results demonstrate that E5-Fc chain binds to Raj 1 cells (Figure 5).

### Example 4

### dAb-Fc fusion pharmacokinetic analysis

For pharmacokinetic analysis 6 groups of three male CD1 mice (age approximately 6 to 7 weeks; body weights approximately 25 to 30g) were injected i.v. into the tail vein with 50µg dAb-Fc (E5-Fc as described in Example 1). The E5-Fc protein was purified from a mammalian cell line as described in Example 2 and dialysed twice for >2h against 500 volumes of phosphate buffered saline. 6 groups of three male CD1 mice (age approximately 6 to 7 weeks; body weights approximately 25 to 30g) were injected i.v. into the tail vein with 50µg dAb (an anti-hen egg lysozyme dAb named HEL-4 which has a C-terminal HA epitope tag, see below for the amino acid sequence). HEL-4 was expressed in the E.coli strain HB2151 and purified from the periplasmic fraction by standard chromatography using protein A and anion exchange. The protein was dialysed twice for >2h against 500 volumes of phosphate buffered saline.

The amino acid sequence of HEL-4.

EVQLLESGGGLVQPGGSLRLSCAASGFRISDEDMGWVRQAPGKGLEWVSSIYGP
SGSTYYADSVKGRFTISRDNSKNTLYLQMNSLRAEDTAVYYCASALEPLSEPLGF
WGQGTLVTVSSAAYPYDVPDYA

At selected time points, 3 animals from each group were humanely killed and a terminal blood sample collected. The blood was allowed to clot (*ca* 30 min) and then centrifuged to prepare serum. The serum was decanted and stored frozen until analysis. To determine the concentrations of dAb or dAb-Fc protein, serum samples were diluted in phosphate buffered saline containing 2% (v/v) Tween-20 and assayed by antigen capture ELISA. For HEL-4 the antigen was hen egg lysozyme that was coated overnight on a Maxisorp plate (Nunc) at 3mg/ml in buffer containing 1.59g/l Na₂CO₃, 2.93g/l NaHCO₃ pH 9.6 at 4°C. For E5-Fc the antigen was β-galactosidase coated overnight on a Maxisorp plate (Nunc) at 10µg/ml in phosphate buffered saline at 4°C. Binding of the HEL-4 and E5-Fc to their respective antigens was detected using an HRP (horse raddish peroxidase) labelled rat monoclonal anti-HA epitope antibody (Roche) or an HRP labelled goat polyclonal anti-human Fc antibody (Sigma), respectively. Known concentrations of HEL-4 and E5-Fc were used to calibrate these readings. Results of the mouse pharmacokinetic experiment (Figure 6 and Table 2) demonstrate much increased half-life in the serum for dAb fused to an Fc region compared with a dAb.

**Table 2**

| | t1/2α (h) | t1/2β (h) | AUC (0-∞) (mg.min/ml) |
|---|---|---|---|
| HEL-4 | 0.067 | 0.34 | 0.1 |
| E5-Fc | 2 | 25.4 | 31.8 |

### Example 5. Efficacy study of TAR1-5-19 in a human TNF transgenic model of arthritis.

As referred to herein **TAR1-5-19** is a Dab which specifically binds to the target human TNF alpha (TAR1).

Tg197 mice are transgenic for the human TNF-globin hybrid gene and heterozygotes at 4-7 weeks of age develop a chronic, progressive polyarthritis with histological features in common with rheumatoid arthritis. [Keffer, J., Probert, L.,Cazlaris, H., Georgopoulos, S.,Kaslaris, E., Kioussis, D., Kollias, G. (1991). Transgenic mice expressing human tumor necrosis factor: a predictive genetic model of arthritis. EMBO J., Vol. 10, pp. 4025-4031.]

To test the efficacy of a V_{κ} dAb Fc fusion (dAb fused to IgG1 CH2-CH3 regions, the dAb being TAR1-5-19) in the prevention of arthritis in the Tg197 model, heterozygous transgenic mice were divided into 5 groups of 10 animals with equal numbers of male and females. Treatment commenced at 3 weeks of age with twice weekly intraperitoneal injections of test items. The treatment groups are listed in Table 1. The control dAb-Fc was a fusion between the Fc region of human IgG1 and an anti-bgalactosidase dAb (termed E5) and was expressed in the supernatant of a stably transfected COS-7 cell line. The TAR1-5-19-Fc fusion was expressed by transient transfection of COS-7. Both Fc fusion proteins were purified by protein A chromatography. TAR1-5-19 monomer was expressed in E.coli and purified by protein L chromatography and IEX. All protein preparations were in phosphate buffered saline and were tested for acceptable levels of endotoxins.

**Table 3. Treatment groups and dosing.**

| Group | Treatment | Twice Weekly Dose |
|---|---|---|
| 1 | Control dAb-Fc Fusion | 10mg/Kg |
| 2 | TAR1-5-19-Fc Fusion | 10mg/Kg |
| 3 | TAR1-5-19-Fc Fusion | 1mg/Kg |
| 4 | TAR1-5-19 monomer | 20mg/Kg |
| 5 | Saline Control | N/A |

The study was performed blind. Each week the animals were weighed and the macrophenotypic signs of arthritis scored according to the following system: 0 = no arthritis (normal appearance and flexion), 1 = mild arthritis (joint distortion), 2 = moderate arthritis (swelling, joint deformation), 3 = heavy arthritis (severely impaired movement). At week 10, the ankle/paw and knee joints of the animals were fixed, embedded and histopathological analysis was performed on the ankle joint using the following system: 0= no detectable pathology, 1= hyperplasia of the synovial membrane and presence of polymorphonuclear infiltrates, 2= pannus and fibrous tissue formation and focal subchondral bone erosion , 3= articular cartilage destruction and bone erosion, 4= extensive articular cartilage destruction and bone erosion. The histology was scored blind.

The outcome of the arthritic scoring clearly demonstrated that 10mg/Kg TAR1-5-19-Fc fusion inhibited the development of arthritis (see Figure 7). A comparison of the median arthritic scores at week 10 of TAR1-5-19-Fc with either the control dAb-Fc, the TAR1-5-19 monomer or the saline control gave a statistically significant effect (P<0.1%). The low dose of TAR1-5-19-Fc did produce a lower median arthritic score than control dAb-Fc however the difference was not statistically significant. There was some evidence (P<5%) of arthritis occurring earlier in the saline group compared with the 1mg/Kg TAR1-5-19-Fc group.

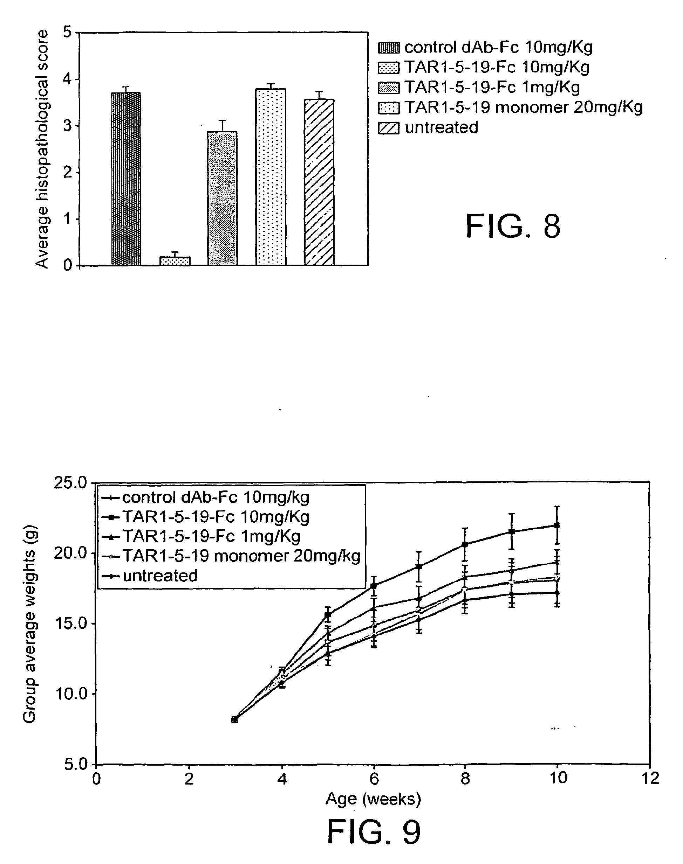

The results from the macrophenotypic scoring of the arthritis in the joints were mirrored in the histopathological scoring (see Figure 8). The prophylactic treatment with a high dose of TAR1-5-19-Fc resulted in a lower histopathological score when compared with the control groups.

Cachexia which is an effect of the increased levels of circulating TNF in the transgenic animals was strongly inhibited by the TAR1-5-19-Fc (high dose) (see Figure 9).

In conclusion, TAR1-5-19-Fc was shown to be a highly effective therapy in the Tg197 model of arthritis.

### Example 6. Expression of a dAb-Fc fusion protein in Pichia pastoris.

### Vector construction

The vector for the methanol inducible, secreted, expression of dAb Fc fusion proteins in Pichia was constructed based on the expression vector pPICZalpha (Invitrogen). The vector was modified to remove the XhoI site at nucleotide 1247 by digestion with XbaI and Kpnl, blunt ending with Pfu polymerase and relegation. The SalI site at nucleotide 1315 was removed by digestion with SalI, blunt ending with Pfu polymerase and relegation. A VK dAb-Fc fusion was then PCR amplified from a mammalian expression construct described above using the primers below and PfuTurbo DNA polymerase (Stratagene):
PVKF2 5'-TCTCTCGAGAAAAGAGACATCCAGATGACCCAGTCTCC-3'
FcPicR1 5'-TAGAATTCTCATCATTTACCCGGAGACAGGGAGA-3'

The PCR product was digested with XhoI and EcoRI, then cloned into EcoRI/XhoI digested expression vector. This gave the construct pPICZalpha-TAR1-5-19Fc which would produce an anti-TNF Fc fusion protein.

To construct a general vector for production of Fc fusions of VH or VK dAbs, the XhoI-NotI dAb fragment was excised from pPICZalpha-TAR1-5-19Fc, and replaced with a XhoI-NotI linker which contained an in frame Sail site (sequence of the fragment, including restriction sites: 5'-CTCGAGAAAAGAGCGTCGACATCTAGATCAGCGGCCGC-3').

Other dAbs could then be cloned into this vector digested with XhoI and NotI after PCR amplification using the following primer pairs and cloned as XhoI NotI fragments.
For VH dAbs :
PVHF1 5'-TCTCTCGAGAAAAGAGAGGTGCAGCTGTTGGAGTCTG-3'
PVHR2 5'-TAGAATTCTTATTAGCTAGAGACGGTGACCAGGGT-3'
For VK dAbs :
PVKF2 5'-TCTCTCGAGAAAAGAGACATCCAGATGACCCAGTCTCC-3'
PVKR1 5'-TAGAATTCTTATTACCGTTTGATTTCCACCTTGGTC-3'

Sequences were verified by sequencing with the following primers
Alpha factor primer (forward): 5'-TACTATTGCCAGCATTGCTGC-3'
3'AOX1 (reverse): 5'-GCAAATGGCATTCTGACATCC-3'

All cloning was performed in E. coli TOP10F' cells. The vectors were linearised with PmeI prior to transformation of Pichia.

This vector, when integrated into the *P. pastoris* genome will express the anti-TNF recombinant dAb-Fc fusion protein TAR1-5-19Fc on induction with methanol. The protein will be produced with an amino terminal yeast alpha mating factor secretion signal, which will direct secretion to the culture medium, during which it will be cleaved off by the Kex2 protease, to leave a homogenous dAb-Fc fusion protein which can be purified from the culture supernatant.

The protein produced here has a Factor Xa protease cleavage site between the dAb and the Fc region. This aids in functional analysis of the protein, but could be replaced by either: a flexible polypeptide linker, a rigid polypeptide linker, or other specific protease cleavable sequence.

The use of a specific protease cleavage site would give advantages in reducing the amount of protein binding non-specifically to an antigen in a non-target tissue which also expressed the chosen protease, where the target tissue did not express. This could be useful in targeted immunotoxins, drug conjugates or prodrug activating enzymes.

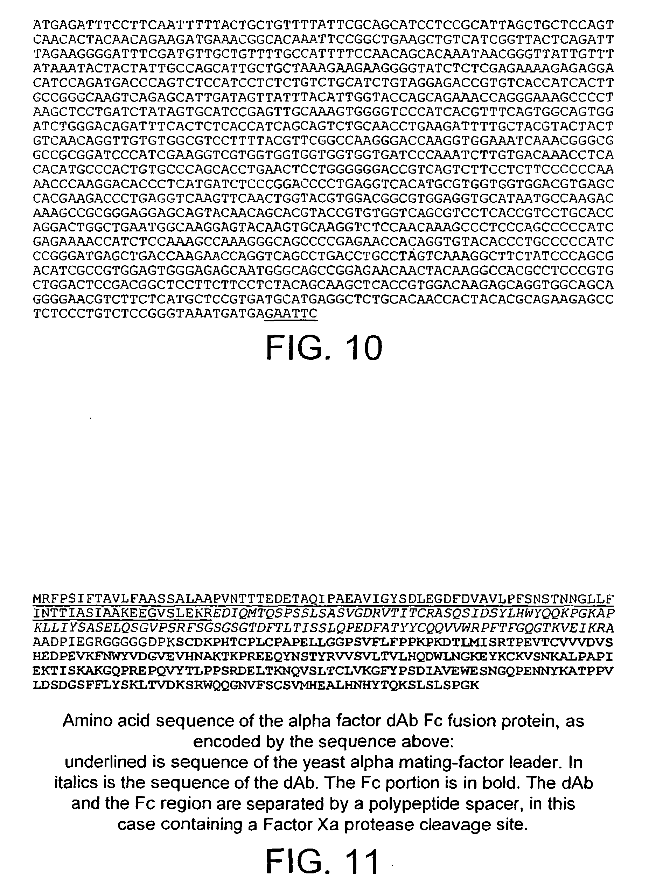

Shown below and in figure 10 is the nucleotide sequence of the alpha factor dAb Fc fusion protein from the start of the alpha factor leader sequence to the EcoRI cloning site.

ATGAGATTTCCTTCAATTTTTACTGCTGTTTTATTCGCAGCATCCTCCGCATTAGCTGCTCCAGT
CAACACTACAACAGAAGATGAAACGGCACAAATTCCGGCTGAAGCTGTCATCGGTTACTCAG
ATTTAGAAGGGGATTTCGATGTTGCTGTTTTGCCATTTTCCAACAGCACAAATAACGGGTTATT
GTTTATAAATACTACTATTGCCAGCATTGCTGCTAAAGAAGAAGGGGTATCTCTCGAGAAAAG
AGAGGACATCCAGATGACCCAGTCTCCATCCTCTCTGTCTGCATCTGTAGGAGACCGTGTCAC
CATCACTTGCCGGGCAAGTCAGAGCATTGATAGTTATTTACATTGGTACCAGCAGAAACCAGG
GAAAGCCCCTAAGCTCCTGATCTATAGTGCATCCGAGTTGCAAAGTGGGGTCCCATCACGTTT
CAGTGGCAGTGGATCTGGGACAGATTTCACTCTCACCATCAGCAGTCTGCAACCTGAAGATTT
TGCTACGTACTACTGTCAACAGGTTGTGTGGCGTCCTTTTACGTTCGGCCAAGGGACCAAGGT
GGAAATCAAACGGGCGGCCGCGGATCCCATCGAAGGTCGTGGTGGTGGTGGTGGTGATCCCA
AATCTTGTGACAAACCTCACACATGCCCACTGTGCCCAGCACCTGAACTCCTGGGGGGACCGT
CAGTCTTCCTCTTCCCCCCAAAACCCAAGGACACCCTCATGATCTCCCGGACCCCTGAGGTCAC
ATGCGTGGTGGTGGACGTGAGCCACGAAGACCCTGAGGTCAAGTTCAACTGGTACGTGGACG
GCGTGGAGGTGCATAATGCCAAGACAAAGCCGCGGGAGGAGCAGTACAACAGCACGTACCGT

GTGGTCAGCGTCCTCACCGTCCTGCACCAGGACTGGCTGAATGGCAAGGAGTACAAGTGCAA
GGTCTCCAACAAAGCCCTCCCAGCCCCCATCGAGAAAACCATCTCCAAAGCCAAAGGGCAGC
CCCGAGAACCACAGGTGTACACCCTGCCCCCATCCCGGGATGAGCTGACCAAGAACCAGGTC
AGCCTGACCTGCCTAGTCAAAGGCTTCTATCCCAGCGACATCGCCGTGGAGTGGGAGAGCAAT
GGGCAGCCGGAGAACAACTACAAGGCCACGCCTCCCGTGCTGGACTCCGACGGCTCCTTCTTC
CTCTACAGCAAGCTCACCGTGGACAAGAGCAGGTGGCAGCAGGGGAACGTCTTCTCATGCTCC
GTGATGCATGAGGCTCTGCACAACCACTACACGCAGAAGAGCCTCTCCCTGTCTCCGGGTAAA
TGATGAGAATTC

The amino acid sequence of the alpha factor dAb Fc fusion protein, as encoded by the nucleotide sequence above is shown below and also in fig 11: underlined is sequence of the yeast alpha mating factor leader. In italics is the sequence of the dAb. The Fc portion is in bold. The dAb and the Fc region are separated by a polypeptide spacer, in this case containing a Factor Xa protease cleavage site.

### Transformation of Pichia pastoris strain KM71H.

Pichia were made competent for electroporation by growing *P. pastoris* KM71H in 0.51 YPD (1% (w/v) yeast extract, 2% (w/v) peptone, 2% (w/v) glucose) medium at 30°C to an OD₆₀₀ₙₘ of 1.0. The cells were then washed twice with ice cold water, once with 20ml ice cold 1M sorbitol, and resuspended in 1ml 1M sorbitol. 80microlitres of the resulting suspension were incubated on ice with 10microlitres of water containing 10micrograms of PmeI linearised vector produced as described above for 5 minutes, and then electroporated in a 0.2cm electrode gap electroporation cuvette at 0.54kV, 25microFarad, with resistance set at infinity in a Biorad gene pulser II with capacitance extender (Biorad). Cells were recovered in 1ml 1M sorbitol, then plated onto YPDS plates (1% (w/v) yeast extract, 2% (w/v) peptone, 2% (w/v) glucose, 1M sorbitol in 1.5% (w/v) agar) supplemented with 100, 500, 1000, or 2000microgram/ml zeocin. Plates were grown for 2-3 days at 30°C, and then colonies were re-streaked to isolate clonal populations. Clones were then characterised for expression levels as described below.

Such a result could also be obtained in other *Pichia pastoris* strains such as X33, or the protease deficient strains smd1163, smd1165 or smd1168 which will be advantageous in reducing proteolytic cleavage of the dAb-Fc fusion protein dusing expression. Alternatively other *Pichia* species such as *Pichia methanolica*, or other yeast and fungal species such as *Hansenula polymorpha, Saccharomyces cerevisiae, Candida boidinii,* or *Aspergillus awamorii*, would be suitable for the expression of dAb Fc fusion proteins.

### Expression

Expression was carried out in baffled shake flasks in complex BMGY medium containing glycerol as a carbon source (1% (w/v) yeast extract, 2% (w/v) peptone, 1% (v/v) glycerol, 1.34% (w/v) yeast nitrogen base, 4x10⁻⁵% (w/v) biotin, 100mM KPO₄ buffer pH6.0). Cultures were grown at 30°C with shaking at 250rpm to an OD₆₀₀ₙₘ of 10, then the pellet was recovered by centrifugation, and resuspended in BMMY to induce expression (1% (w/v) yeast extract, 2% (w/v) peptone, 0.5% (v/v) methanol, 1.34% (w/v) yeast nitrogen base, 4x10⁻⁵% (w/v) biotin, 100mM KPO₄ buffer pH6.0). Peak expression levels of 30milligram/l were observed between 24-48hr post induction at 30°C.

Growth and expression could also be performed in other media including minimal or chemically defined medium, as well as in complex media, with equivalent results. Growth to higher cell densities under conditions of controlled carbon source feeding, controlled methanol induction levels and controlled oxygen levels in a fermenter using fed batch or continuous processes, would lead to higher expression levels.

If a glycosylation pattern is required that is closer to that seen in humans, mammalian like glycosylation could be obtained using modifications of the glycosylation enzymes in Pichia, such as that described in: Hamilton SR, Bobrowicz P, Bobrowicz B, Davidson RC, Li H, Mitchell T, Nett JH, Rausch S, Stadheim TA, Wischnewski H, Wildt S, Gemgross TU (2003). Production of complex human glycoproteins in yeast. Science. 29;301(5637):1171. This would yield a homogenously glycosylated product.

### Purification.

Purification was carried out on supernatant clarified by centrifugation at 2000xg for 20 min at 4C. Supernatant was loaded at 300cm/hr onto a 20cm deep bed of Streamline Protein A matrix (Amersham Biotech). After loading, unbound material was removed by washing with PBS supplemented with 0.35M NaCl. The fusion protein was eluted with 0.1M glycine, 0.15M NaCl, pH 3.0. Fractions were neutralised with 0.2 volumes 1M Tris-HCl, pH 8.0. Pure dAb-Fc fusion was further purified from this material by ion exchange chromatography on a 5ml Resource Q column (Amersham Biotech) 20mM Tris-HCl buffer at pH8.5 using a 0 to 0.5M NaCl gradient over 30 column volumes.

### Analysis.

### Results are shown in figure 13.

Amino terminal sequencing showed that the protein had been processed as predicted by the *P. pastoris* Kex2 protease to give the amino-terminal sequence of NH₂-EDQIM after 5 cycles of Edman degradation.

Non-reduced and reduced SDS-PAGE analysis (Figure 13) showed that the protein was the same size as that produced in mammalian cells using the same TAR1-5-19-Fc fusion protein construct in a mammalian expression vector.

>75% of the Fc homodimers were linked by inter-chain disulphide bonds, while <25% were not disulphide linked. This is similar to the situation seen in mammalian cells, where a portion of Fc fusion proteins exist as non-disulphide linker homodimers.

Gel filtration analysis on a Superdex 75 column (Amersham Biotech) gave the predicted molecular weight of 102.4kDa, as predicted for a glycosylated homodimer.

### Antigen binding activity

Results are shown in figure 12.

Antigen binding activity was determined using a TNF receptor binding assay (fig 12). A 96 well Nunc Maxisorp plate is coated with a mouse anti-human Fc antibody, blocked with 1% BSA, then TNF receptor 1-Fc fusion is added. The dAb-Fc fusion protein at various concentrations is mixed with 10ng/ml TNF protein and incubated at room temperature for >1hour. This mixture is added to the TNF receptor 1-Fc fusion protein coated plates, and incubated for 1hour at room temperature. The plates are then washed to remove unbound free dAb-Fc fusion, TNF and dAb-Fc/TNF complexes. The plate was then incubated sequentially with a biotinylated anti-TNF antibody and streptavidin-horse radish peroxidase. The plate was then incubated with the chromogenic horse radish peroxidase substrate TMB. The colour development was stopped with the addition of 1M hydrochloric acid, and absorbance read at 450nm. The absorbance read is proportional to the amount of TNF bound, hence, the TAR1-5-19Fc fusion protein will compete with the TNF receptor for binding of the TNF, and reduce the signal in the assay.

The P. pastoris produced protein had an equivalent activity to the mammalian protein in the vitro TNF receptor assay described above.

### Complement activation activity

The protein produced was effective at activation of human complement after antigen binding, as measured by the following assay:

96-well Maxisop plates (Nunc) were coated with human TNF at 1microgram/ml. The dAb-Fc fusion or control antibody was bound to the TNF coated plates, which were washed with phosphate buffered saline to remove unbound antibody, then pre-incubated with human complement C1 at 1microgram/ml (Merck Biosciences, consisting of a complex of the stoichiomety: (C1r)₂ (C1s)₂ C1q) in complement fixation diluent buffer (Oxoid, 0.575g/l barbitone, 8.5g/l NaCl, 0.168g/l MgCl₂, 0.028g/l CaCl₂, 0.185g/l barbitone soluble, pH 7.2), for 30minutes, after which the substrate Methoxycarbonyl-Lys (z) -Gly-Arg-pNA (Bachem) was added to a final concentration of 2. 5mM. This is cleaved by activated C1s to release pNA, and the assay followed by colour development at 405nm due to release of pNA.

At 82.5microgram/ml the dAb-Fc fusion gave an absorbance at 405nm of 0.09AU above background after 180mins.

In situations where complement activation is important for dAb-Fc fusion functionality, such as complement lysis of target tumour cells, this activity is advantageous. If the Fc fusion is for other reasons, where complement activation is not required or is deleterious to function, removal of the glycosylated Asparagine residue would remove the glycosylation site, and a homogenous aglycosyl protein could be produced.

## Claims

1. A method for synthesising a single-domain-effector group (dAb-effector group) suitable for *in vivo* use comprising the steps of :
(a) selecting an antibody single variable domain having an epitope binding specificity; and
(b) attaching the single domain of step (a) to one or more antibody constant regions and/or hinge region (an effector group),
wherein the antibody single variable domain is a light chain variable domain.

2. A method according to claim 1, wherein the light chain variable domain is a member of the V_{κ} sub-group of domains.

3. A method according to claim 1, wherein the light chain variable domain is a member of the Vλ sub-group of domains.

4. A method according to any preceding claim, wherein the effector group comprises any one or more of those groups selected from the group consisting of: an antibody light chain constant region (C_{L}), an antibody CH1 heavy chain domain, an antibody CH2 heavy chain domain, an antibody CH2 heavy chain domain, an antibody CH3 heavy chain domain, an Fc region of an antibody and a hinge region of an antibody molecule.

5. A method according to any preceding claim, wherein the effector group constitutes an Fc region of an antibody.

6. A method according to claim 5, wherein the effector group consists of a CH2 and CH3 domain.

7. A method according to claim 5, wherein the effector group consists of a CH2 domain, a CH3 domain and the hinge region of an antibody molecule.

8. A method according to any preceding claim, wherein the antibody single variable domain is a non-Camelid variable domain.

9. A method according to any preceding claim, wherein the antibody single variable domain comprises one or more human framework regions.

10. A method according to any preceding claim, wherein the antibody single variable domain comprises four framework regions as defined by Kabat, which are derived from a human.

11. A method according to claim 9 or claim 10, wherein one or more of the human framework regions are defined by Kabat are identical on the amino acid level to those encoded by human germline antibody genes.

12. A method according to any preceding claim, wherein the antibody single variable domain is a human variable domain.

13. A method according to any preceding claim, wherein the antibody single variable domain is not created in an animal.

14. A method according to any preceding claim, wherein the antibody single variable domain binds to the superantigen protein L.

15. A method according to any preceding claim, wherein the effector group is of Camelid or human origin.

16. A method according to any preceding claim, wherein the single variable domain comprises one or more human framework regions and the immunoglobulin effector group is of human origin.

17. A method according to claim 16, wherein the single variable domain comprises four human framework regions and the immunoglobulin effector group is of human origin.

18. A method according to any preceding claim, wherein attaching of the single variable domain to the effector group in step (b) is effected by expressing the single-domain-effector group as a fusion polypeptide.

19. A dAb-effector group suitable for *in vivo* use which comprises an antibody single variable domain having an epitope binding specificity attached to one or more antibody constant regions and/or hinge region (an effector group), wherein the antibody single variable domain is a light chain variable domain.

20. A dAb-effector group for use as a medicament, wherein the dAb is a light chain single variable domain and the effector group is one or more antibody constant regions and/or hinge region.

21. A dAb-effector group according to claim 20, wherein the light chain variable domain is a member of the V_{κ} sub-group of domains.

22. A dAb-effector group according to claim 20, wherein the light chain variable domain is a member of the Vλ sub-group of domains.

23. A dAb-effector group according to claims 19 to 22, wherein the effector group comprises any one or more of those groups selected from the group consisting of: an antibody light chain constant region (C_{L}) and antibody CH1 heavy chain domain, and antibody CH2 heavy chain domain, an antibody CH3 heavy chain domain, a Fc region of an antibody and a hinge region of an antibody molecule.

24. A dAb-effector group according to claim 23, wherein the effector group consists of a CH2 and CH3 domain.

25. A dAb-effector group according to claim 23, wherein the effector group consists of a CH2 domain, a CH3 domain and the hinge region of an antibody molecule.

26. A dAb-effector group according to claims 20 to 23, wherein the effector group constitutes an Fc region of an antibody.

27. A dAb-effector group according to any of claims 19 to 26, wherein the antibody single variable domain comprises human framework regions.

28. A dAb-effector group according to any of claims 19 to 27, wherein the antibody single chain variable domain is of human origin.

29. A dAb-effector group according to any one of claims 19 to 28, wherein the effector group is of Camelid or human origin.

30. A dAb-effector group according to any of claims 19 to 29 wherein the single variable domain comprises one or more human framework regions and the immunoglobulin effector group is of human origin.

31. Two or more dAb-effector groups comprising at least one dAb-effector group according to any one or claims 20 to 31 provided as a higher order structure selected from the group consisting of the following: dimers, trimers and multimers.

32. Two dAb-effector groups according to claim 31 provided as a heterodimer or a homodimer.

33. Two dAb-effector groups according to claim 32 provided as a homodimer.

34. A composition comprising a dAb-effector group, for use as a medicament, wherein the dAb-effector group is described by any of those structures shown in Figure 1(b) and (g), wherein 1=VL and VL is a VL dAb.

35. A composition comprising a dimer of dAb-effector groups as described in claim 34.

36. A nucleic acid molecule encoding at least a dAb-effector group according to any of claims 19 to 35.

37. A nucleic acid molecule according to claim 36 further encoding a signal sequence for export of the dAb and effector group from the cytoplasm of a host cell upon expression.

38. A vector comprising nucleic acid according to claim 36 or claim 37.

39. A host cell transfected with a vector according to claim 38.

40. A composition comprising a dAb-effector group(s) according to any one of claims 19 to 35 and a pharmaceutically acceptable carrier, diluent or excipient.

41. A composition according to claim 40 having a t½ alpha of 15 minutes or more.

42. A composition according to claim 40 having a t½ alpha from 1 to 6 hours.

43. A composition according to claim 40, 41 or 42 having a t½ beta of 2.5 hours or more.

44. A composition according to claim 43 having a t½ beta of 1 day or more.

45. A composition according to claim 43 having a t½ beta of 2 days or more.

46. A composition according to claim 43 having a t½ beta of 3 days or more.

47. A composition according to any one of claims 40 to 46 having an AUC of 1 mg.min/ml or more.

48. A composition according to claim 47 having an AUC from 15 to 150 mg.min/ml.

49. The use of a dAb-effector group according to any of claims 19 to 33, or a composition according to any one of claims 35 to 36, in the preparation of a medicament for the treatment and/or prevention of disease; wherein the antibody single variable domain (dAb) is a light chain variable domain.

50. The use of a dAb-effector group according to any of claims 19 to 33, or a composition according to any one of claims 35 to 35, in the preparation of a medicament for the prophylaxis and/or prevention of an inflammatory disease in a patient; wherein the antibody single variable domain (dAb) is a light chain variable domain.

51. The use according to claim 50 wherein the inflammatory disease is mediated by TNF alpha and is selected from the group consisting of the following: rhematoid arthritis, psoriasis, Crohn's disease, inflammatory bowel disease (BD), multiple sclerosis, septic shock, Alzheimer's, coronary thrombosis, chronic obstructive pulmonary disease (COPD) and glomerular nephritis.

52. The use according to claim 51, wherein the inflammatory disease is rheumatoid arthritis.

53. The use according to claim 52, wherein the antibody light chain single variable domain (dAb) specifically binds to TNF alpha.

54. The use of a dAb-effector group according to any of claims 19 to 33 or a composition according to claim 34 or 35 in the preparation of a medicament for reducing and/or preventing and/or suppressing cachexia in patient.

55. A use according to claim 54 wherein the cachexia is mediated human TNF alpha and the patient is a human.

56. A use according to claim 54-55 wherein the effector group is Fc.

57. A use according to any of claims 54 to 56 wherein the dAb-effector group is administered in a dosage range of 0.5 to 20mg/Kg.

58. A use according to claim 57, wherein the dAb-effector group is administered in a dose of range of 1 to 10mg/Kg.

59. A composition according to claim 34 having the structure depicted in Figure 1(g)(ii) wherein the single variable domains V_{H} and V_{L} each form a respective epitope or antigen binding site and the epitope or antigen specificity of the single variable domains is different.

## Patentansprüche

1. Verfahren zur Synthese einer Einzeldomänen-Effektorgruppe (dAb-Effektorgruppe), die für eine *in vivo* Verwendung geeignet ist, bei dem man: (a) eine einzelne variable Antikörper-Domäne auswählt, die eine Bindungsspezifität für ein Epitop aufweist, und (b) die einzelne Domäne aus Schritt (a) mit einer oder mit mehreren konstanten Regionen und/oder einer Hinge-Region (einer Effektorgruppe) eines Antikörpers verknüpft, wobei die einzelne variable Antikörper-Domäne eine variable Domäne der leichten Kette ist.

2. Verfahren nach Anspruch 1, wobei die variable Domäne der leichten Kette ein Mitglied der Domänenuntergruppe V_{κ} ist.

3. Verfahren nach Anspruch 1, wobei die variable Domäne der leichten Kette ein Mitglied der Domänenuntergruppe Vλ ist.

4. Verfahren nach einem der vorhergehenden Ansprüche, wobei die Effektorgruppe eine oder mehrere von den Gruppen umfasst, die ausgewählt sind aus der Gruppe bestehend aus: einer konstanten Region der leichte Kette eines Antikörpers (C_{L}), einer CH1-Domäne der schweren Kette eines Antikörpers, einer CH2-Domäne der schweren Kette eines Antikörpers, einer CH3-Domäne der schweren Kette eines Antikörpers, einer Fc-Region eines Antikörpers und einer Hinge-Region eines Antikörpermoleküls.

5. Verfahren nach einem der vorhergehenden Ansprüche, wobei die Effektorgruppe eine Fc-Region eines Antikörpers bildet.

6. Verfahren nach Anspruch 5, wobei die Effektorgruppe aus einer CH2- und einer CH3-Domäne besteht.

7. Verfahren nach Anspruch 5, wobei die Effektorgruppe aus einer CH2-Domäne, einer CH3-Domäne und der Hinge-Region eines Antikörpermoleküls besteht.

8. Verfahren nach einem der vorhergehenden Ansprüche, wobei die einzelne variable Antikörper-Domäne eine variable nicht-Kamelid-Domäne ist.

9. Verfahren nach einem der vorhergehenden Ansprüche, wobei die einzelne variable Antikörper-Domäne eine oder mehrere humane Gerüstregionen umfasst.

10. Verfahren nach einem der vorhergehenden Ansprüche, wobei die einzelne variable Antikörper-Domäne vier nach Kabat definierte Gerüstregionen umfasst, die aus einem Menschen abgeleitet sind.

11. Verfahren nach Anspruch 9 oder 10, wobei eine oder mehrere der nach Kabat definierten humanen Gerüstregionen auf der Aminsäureebene identisch zu solchen sind, die von Antikörper-Genen der humanen Keimbahn kodiert werden.

12. Verfahren nach einem der vorhergehenden Ansprüche, wobei die einzelne variable Domäne des Antikörpers eine humane variable Domäne ist.

13. Verfahren nach einem der vorhergehenden Ansprüche, wobei die einzelne variable Antikörper-Domäne nicht in einem Tier hergestellt wird.

14. Verfahren nach einem der vorhergehenden Ansprüche, wobei die einzelne variable Antikörper-Domäne an das Superantigenprotein L bindet.

15. Verfahren nach einem der vorhergehenden Ansprüche, wobei die Effektorgruppe kameliden oder humanen Ursprungs ist.

16. Verfahren nach einem der vorhergehenden Ansprüche, wobei die einzelne variable Domäne eine oder mehrere humane Gerüstregionen umfasst, und die Immunglobulin-Effektorgruppe humanen Ursprungs ist.

17. Verfahren nach Anspruch 16, wobei die einzelne variable Domäne vier humane Gerüstregionen umfasst und die Immunglobulin-Effektorgruppe humanen Ursprungs ist.

18. Verfahren nach einem der vorhergehenden Ansprüche, wobei die Bindung der einzelnen variablen Domäne an die Effektorgruppe in Schritt (b) beeinflusst wird, indem man die Einzeldomänen-Effektorgruppe als Fusionspolypeptid exprimiert.

19. dAb-Effektorgruppe, die für eine *in vivo* Verwendung geeignet ist, und die eine einzelne variable Antikörper-Domäne mit einer Bindungsspezifität für ein Epitop umfasst, welche mit einer oder mehreren konstanten Regionen und/oder einer Hinge-Region (einer Effektorgruppe) eines Antikörpers verknüpft ist, wobei die einzelne variable Antikörper-Domäne eine variable Domäne der leichten Kette ist.

20. dAb-Effektorgruppe zur Verwendung als Medikament, wobei dAb eine einzelne variable Domäne der leichten Kette ist, und die Effektorgruppe eine oder mehrere konstante Regionen und/oder eine Hinge-Region eines Antikörpers ist.

21. dAb-Effektorgruppe nach Anspruch 20, wobei die variable Domäne der leichten Kette ein Mitglied der Domänenuntergruppe V_{κ} ist.

22. dAb-Effektorgruppe nach Anspruch 20, wobei die variable Domäne der leichten Kette ein Mitglied der Domänenuntergruppe V_{λ} ist.

23. dAb-Effektorgruppe nach Anspruch 19-22, wobei die Effektorgruppe eine oder mehrere Gruppen umfasst, die ausgewählt sind aus der Gruppe bestehend aus: einer konstanten Region der leichten Kette (C_{L}) eines Antikörpers, einer CH1-Domäne der schweren Kette eines Antikörpers, einer CH2-Domäne der schweren Kette eines Antikörpers, einer CH3-Domäne der schweren Kette eines Antikörpers, einer Fc-Region eines Antikörpers und einer Hinge-Region eines Antikörper-Moleküls.

24. dAb-Effektorgruppe nach Anspruch 23, wobei die Effektorgruppe aus einer CH2- und einer CH3-Domäne besteht.

25. dAb-Effektorgruppe nach Anspruch 23, wobei die Effektorgruppe aus einer CH2-Domäne, einer CH3-Domäne und der Hinge-Region eines Antikörpermoleküls besteht.

26. dAb-Effektorgruppe nach den Ansprüchen 20-23, wobei die Effektorgruppe eine Fc-Region eines Antikörpers bildet.

27. dAb-Effektorgruppe nach einem der Ansprüche 19 bis 26, wobei die einzelne variable Antikörper-Domäne aus humanen Gerüstregionen besteht.

28. dAb-Effektorgruppe nach einem der Ansprüche 19 bis 27, wobei die einzelkettige variable Antikörper-Domäne humanen Ursprungs ist.

29. dAb-Effektorgruppe nach einem der Ansprüche 19 bis 28, wobei die Effektorgruppe kameliden oder humanen Ursprungs ist.

30. dAb-Effektorgruppe nach einem der Ansprüche 19 bis 29, wobei die einzelne variable Domäne eine oder mehrere humane Gerüstregionen umfasst und die Immunglobulin-Effektorgruppe humanen Ursprungs ist.

31. Zwei oder mehrere dAb-Effektorgruppen, die mindestens eine dAb-Effektorgruppe nach einem der Ansprüche 20-31 umfasst, wobei diese als Struktur höherer Ordnung bereitgestellt werden, die ausgewählt ist aus der Gruppe bestehend aus: Dimeren, Trimeren und Multimeren.

32. Zwei dAb-Effektorgruppen nach Anspruch 31, die als Heterodimer oder Homodimer bereitgestellt werden.

33. Zwei dAb-Effektorgruppen nach Anspruch 32, die als Homodimer bereitgestellt werden.

34. Zusammensetzung, die eine dAb-Effektorgruppe umfasst, zur Verwendung als Medikament, wobei die dAb-Effektorgruppe als eine beliebige der Strukturen beschrieben wird, welche in Figur 1 (b) und (g) dargestellt sind, wobei 1=VL ist und VL eine VL dAb ist.

35. Zusammensetzung, die ein Dimer aus dAb-Effektorgruppen umfasst, wie es in Anspruch 34 beschrieben ist.

36. Nukleinsäuremolekül, das für mindestens eine dAb-Effektorgruppe nach einem der Ansprüche 19-35 kodiert.

37. Nukleinsäuremolekül nach Anspruch 36, das ferner für eine Signalsequenz kodiert, welche bei Expression für den Export der dAb und der Effektorgruppe aus dem Zytoplasma der Wirtszelle sorgt.

38. Vektor, der eine Nukleinsäure nach Anspruch 36 oder nach Anspruch 37 umfasst.

39. Wirtszelle, die mit einem Vektor nach Anspruch 38 transfiziert ist.

40. Zusammensetzung, die (eine) dAb-Effektorgruppe(n) nach einem der Ansprüche 19 bis 35 und einen pharmazeutisch akzeptablen Träger, ein Verdünnungsmittel oder einen Hilfsstoff umfasst.

41. Zusammensetzung nach Anspruch 40, die eine t½ alpha von 15 Minuten oder mehr aufweist.

42. Zusammensetzung nach Anspruch 40, die eine t½ alpha von 1 bis 6 Stunden aufweist.

43. Zusammensetzung nach Anspruch 40, 41 oder 42, die eine t½ alpha von 2, 5 Stunden oder mehr aufweist.

44. Zusammensetzung nach Anspruch 43, die eine t½ beta von einem Tag oder mehr aufweist.

45. Zusammensetzung nach Anspruch 43, die eine t½ beta von 2 Tagen oder mehr aufweist.

46. Zusammensetzung nach Anspruch 43, die eine t½ beta von 3 Tagen oder mehr aufweist.

47. Zusammensetzung nach einem der Ansprüche 40 bis 46, die eine AUC von 1 mg.min/ml oder mehr aufweist.

48. Zusammensetzung nach Anspruch 47, die eine AUC von 15-150 mg.min/ml aufweist.

49. Verwendung einer dAb-Effektorgruppe nach einem der Ansprüche 19 bis 33 oder einer Zusammensetzung nach einem der Ansprüche 35 bis 36 bei der Herstellung eines Medikaments zur Behandlung und/oder Vermeidung einer Krankheit, wobei die einzelne variable Antikörper-Domäne (dAb) eine variable Domäne der leichten Kette ist.

50. Verwendung einer dAb-Effektorgruppe nach einem der Ansprüche 19 bis 33 oder eine Zusammensetzung nach einem der Ansprüche 34 bis 35 bei der Herstellung eines Medikaments zur Prophylaxe und/oder Behandlung einer entzündlichen Erkrankung in einem Patienten, wobei die einzelne variable Antikörper-Domäne (dAb) des Antikörpers eine variable Domäne der leichten Kette ist.

51. Verwendung nach Anspruch 50, wobei die entzündliche Erkrankung durch TNF alpha vermittelt wird und ausgewählt ist aus der Gruppe, die aus folgendem besteht: rheumatoide Arthritis, Psoriasis, Morbus Crohn, entzündliche Darmkrankheit (BD), multiple Sklerose, septischer Schock, Alzheimer, Koronarthrombose, chronisch obstruktive Lungenkrankheit (chronic obstructive pulmonary disease, COPD) und glomeruläre Nephritis.

52. Verwendung nach Anspruch 51, wobei die entzündliche Erkrankung rheumatoide Arthritis ist.

53. Verwendung nach Anspruch 52, wobei die einzelne variable Antikörper-Domäne (dAb) der leichten Kette spezifisch an TNF alpha bindet.

54. Verwendung einer dAb-Effektorgruppe nach einem der Ansprüche 19 bis 33 oder einer Zusammensetzung nach den Ansprüchen 34 oder 35 zur Herstellung eines Medikaments zur Verringerung und/oder Vermeidung und/oder Unterdrückung von Kachexie in einem Patienten.

55. Verwendung nach Anspruch 54, wobei die Kachexie durch humanes TNF alpha vermittelt wird und der Patient ein Mensch ist.

56. Verwendung nach Anspruch 54 bis 55, wobei die Effektorgruppe Fc ist.

57. Verwendung nach einem der Ansprüche 54 bis 56, wobei die dAb-Effektorgruppe in einem Dosierungsbereich von 0,5 bis 20 mg/kg verabreicht wird.

58. Verwendung nach Anspruch 57, wobei die dAb-Effektorgruppe in einem Dosierungsbereich von 1 bis 10 mg/kg verabreicht wird.

59. Zusammensetzung nach Anspruch 34, welche die in Figur 1 (g) (ii) dargestellte Struktur aufweist, wobei die einzelnen variablen Domänen V_{H} und V_{L} jeweils ein entsprechendes Epitop oder eine entsprechende Antigenbindungsstelle bilden, wobei sich die Epitop- oder Antigenspezifität der einzelnen variablen Domänen unterscheiden.

## Revendications

1. Procédé de synthèse d'un groupe effecteur à domaine simple (groupe effecteur - dAb) approprié pour une utilisation *in vivo* comprenant les étapes consistant à :
(a) choisir un domaine variable simple d'anticorps ayant une spécificité de liaison à un épitope ; et
(b) lier le domaine simple de l'étape (a) à une ou plusieurs régions constantes et/ou régions charnières de l'anticorps (un groupe effecteur),
dans lequel le domaine variable simple d'anticorps est un domaine variable de la chaîne légère.

2. Procédé selon la revendication 1, dans lequel le domaine variable de la chaîne légère est un élément du sous-groupe de domaines V_{κ}.

3. Procédé selon la revendication 1, dans lequel le domaine variable de la chaîne légère est un élément du sous-groupe de domaines V_{λ}.

4. Procédé selon l'une quelconque des revendications précédentes, dans lequel le groupe effecteur comprend l'un quelconque ou plusieurs des éléments de ces groupes choisis dans le groupe comprenant : une région constante (C_{L}) de la chaîne légère d'anticorps, un domaine CH1 de la chaîne lourde d'anticorps, un domaine CH2 de la chaîne lourde d'anticorps, un domaine CH2 de la chaîne lourde d'anticorps, un domaine CH3 de la chaîne lourde d'anticorps, une région Fc d'un anticorps et une région charnière d'une molécule d'anticorps.

5. Procédé selon l'une quelconque des revendications précédentes, dans lequel le groupe effecteur constitue une région Fc d'un anticorps.

6. Procédé selon la revendication 5, dans lequel le groupe effecteur consiste en un domaine CH2 et CH3.

7. Procédé selon la revendication 5, dans lequel le groupe effecteur consiste en un domaine CH2, un domaine CH3 et la région charnière d'une molécule d'anticorps.

8. Procédé selon l'une quelconque des revendications précédentes, dans lequel le domaine variable simple d'anticorps est un domaine variable n'appartenant pas à un camélidé.

9. Procédé selon l'une quelconque des revendications précédentes, dans lequel le domaine variable simple d'anticorps comprend une ou plusieurs régions charpentes humaines.

10. Procédé selon l'une quelconque des revendications précédentes, dans lequel le domaine variable simple d'anticorps comprend quatre régions charpentes telles que définies par Kabat, qui sont dérivées d'un humain.

11. Procédé selon la revendication 9 ou la revendication 10, dans lequel la ou les régions charpentes humaines telles que définies par Kabat sont identiques en ce qui concerne les acides aminés à celles codées par des gènes d'anticorps de la lignée germinale humaine.

12. Procédé selon l'une quelconque des revendications précédentes, dans lequel le domaine variable simple d'anticorps est un domaine variable humain.

13. Procédé selon l'une quelconque des revendications précédentes, dans lequel le domaine variable simple d'anticorps n'est pas créé dans un animal.

14. Procédé selon l'une quelconque des revendications précédentes, dans lequel le domaine variable simple d'anticorps se lie au super-antigène qu'est la protéine L.

15. Procédé selon l'une quelconque des revendications précédentes, dans lequel le groupe effecteur est d'origine humaine ou provient d'un camélidé.

16. Procédé selon l'une quelconque des revendications précédentes, dans lequel le domaine variable simple comprend une ou plusieurs régions charpentes humaines et le groupe effecteur de type immunoglobuline est d'origine humaine.

17. Procédé selon la revendication 16, dans lequel le domaine variable simple comprend quatre régions charpentes humaines et le groupe effecteur de type immunoglobuline est d'origine humaine.

18. Procédé selon l'une quelconque des revendications précédentes, dans lequel l'étape consistant à lier le domaine variable simple au groupe effecteur (étape (b)) est effectuée en exprimant le groupe effecteur à domaine simple sous la forme d'un polypeptide de fusion.

19. Groupe effecteur - dAb approprié pour une utilisation *in vivo* qui comprend un domaine variable simple d'anticorps ayant une spécificité de liaison à un épitope lié à une ou plusieurs régions constantes et/ou régions charnières d'anticorps (un groupe effecteur), dans lequel le domaine variable simple d'anticorps est un domaine variable à chaîne légère.

20. Groupe effecteur - dAb destiné à être utilisé comme médicament, dans lequel le dAb représente un domaine variable simple à chaîne légère et le groupe effecteur représente une ou plusieurs régions constantes et/ou régions charnières d'anticorps.

21. Groupe effecteur - dAb selon la revendication 20, dans lequel le domaine variable à chaîne légère est un élément du sous-groupe de domaines Vκ.

22. Groupe effecteur - dAb selon la revendication 20, dans lequel le domaine variable à chaîne légère est un élément du sous-groupe de domaines Vλ.

23. Groupe effecteur - dAb selon l'une quelconque des revendications 19 à 22, dans lequel le groupe effecteur comprend l'un quelconque ou plusieurs des éléments de ces groupes choisis dans le groupe comprenant : une région constante (C_{L}) de la chaîne légère d'anticorps et un domaine CH1 de la chaîne lourde d'anticorps, un domaine CH2 de la chaîne lourde d'anticorps, un domaine CH3 de la chaîne lourde d'anticorps, une région Fc d'un anticorps et une région charnière d'une molécule d'anticorps.

24. Groupe effecteur - dAb selon la revendication 23, dans lequel le groupe effecteur consiste en un domaine CH2 et CH3.

25. Groupe effecteur - dAb selon la revendication 23, dans lequel le groupe effecteur consiste en un domaine CH2, un domaine CH3 et la région charnière d'une molécule d'anticorps.

26. Groupe effecteur - dAb selon l'une quelconque des revendications 20 à 23, dans lequel le groupe effecteur se compose d'une région Fc d'un anticorps.

27. Groupe effecteur - dAb selon l'une quelconque des revendications 19 à 26, dans lequel le domaine variable simple d'anticorps comprend des régions charpentes humaines.

28. Groupe effecteur - dAb selon l'une quelconque des revendications 19 à 27, dans lequel le domaine variable simple d'anticorps est d'origine humaine.

29. Groupe effecteur - dAb selon l'une quelconque des revendications 19 à 28, dans lequel le groupe effecteur est d'origine humaine ou provient d'un camélidé.

30. Groupe effecteur - dAb selon l'une quelconque des revendications 19 à 29, dans lequel le domaine variable simple comprend une ou plusieurs régions charpentes humaines et le groupe effecteur d'immunoglobuline est d'origine humaine.

31. Deux ou plusieurs groupes effecteurs - dAb comprenant au moins un groupe effecteur - dAb selon l'une quelconque des revendications 20 à 31 fournis sous la forme d'une structure d'ordre supérieur choisie dans le groupe comprenant ce qui suit : des dimères, des trimères et des multimères.

32. Deux groupes effecteurs - dAb selon la revendication 31 fournis sous la forme d'un hétérodimère ou d'un homodimère.

33. Deux groupes effecteurs - dAb selon la revendication 32 fournis sous la forme d'un homodimère.

34. Composition comprenant un groupe effecteur - dAb, destinée à être utilisée comme médicament, dans laquelle le groupe effecteur - dAb est décrit par l'une quelconque des structures montrées sur la figure 1(b) et (g), dans lesquelles 1 = VL et VL représente un dAb à VL.

35. Composition comprenant un dimère de groupes effecteurs - dAb tels que décrits dans la revendication 34.

36. Molécule d'acide nucléique codant pour au moins un groupe effecteur - dAb selon l'une quelconque des revendications 19 à 35.

37. Molécule d'acide nucléique selon la revendication 36 codant en outre pour une séquence signal destinée à exporter le dAb et le groupe effecteur hors du cytoplasme d'une cellule hôte après expression.

38. Vecteur comprenant un acide nucléique selon la revendication 36 ou la revendication 37.

39. Cellule hôte transfectée avec un vecteur selon la revendication 38.

40. Composition comprenant un(des) groupe(s) effecteur(s)-dAb selon l'une quelconque des revendications 19 à 35 et un transporteur, un diluant ou un excipient pharmaceutiquement acceptables.

41. Composition selon la revendication 40 ayant un t½ alpha supérieur ou égal à 15 minutes.

42. Composition selon la revendication 40 ayant un t½ alpha allant de 1 à 6 heures.

43. Composition selon la revendication 40, 41 ou 42 ayant un t½ bêta supérieur ou égal à 2,5 heures.

44. Composition selon la revendication 43 ayant un t½ bêta supérieur ou égal à 1 jour.

45. Composition selon la revendication 43 ayant un t½ bêta supérieur ou égal à 2 jours.

46. Composition selon la revendication 43 ayant un t% bêta supérieur ou égal à 3 jours.

47. Composition selon l'une quelconque des revendications 40 à 46 ayant une ASC supérieure ou égale à 1 mg.min/ml.

48. Composition selon la revendication 47 ayant une ASC allant de 15 à 150 mg.min/ml.

49. Utilisation d'un groupe effecteur - dAb selon l'une quelconque des revendications 19 à 33, ou d'une composition selon l'une quelconque des revendications 35 à 36, dans la préparation d'un médicament destiné au traitement et/ou à la prévention de maladies, dans lequel le domaine variable simple d'anticorps (dAb) est un domaine variable de la chaîne légère.

50. Utilisation d'un groupe effecteur - dAb selon l'une quelconque des revendications 19 à 33, ou d'une composition selon l'une quelconque des revendications 34 à 35, dans la préparation d'un médicament destiné à la prophylaxie et/ou au traitement d'une maladie inflammatoire chez un patient, dans lequel le domaine variable simple d'anticorps (dAb) est un domaine variable de la chaîne légère.

51. Utilisation selon la revendication 50 dans laquelle la maladie inflammatoire est induite par le TNF alpha et est choisie dans le groupe comprenant ce qui suit : l'arthrite rhumatoïde, le psoriasis, la maladie de Crohn, une maladie intestinale inflammatoire (IBD), la sclérose en plaques, le choc septique, la maladie d'Alzheimer, la thrombose coronaire, une maladie pulmonaire obstructive chronique (BPCO) et une glomérulonéphrite.

52. Utilisation selon la revendication 51, dans laquelle la maladie inflammatoire est l'arthrite rhumatoïde.

53. Utilisation selon la revendication 52, dans laquelle le domaine variable simple de chaîne légère d'anticorps (dAb) se lie spécifiquement au TNF alpha.

54. Utilisation d'un groupe effecteur - dAb selon l'une quelconque des revendications 19 à 33 ou d'une composition selon la revendication 34 ou 35 dans la préparation d'un médicament destiné à réduire et/ou à prévenir et/ou à supprimer la cachexie chez un patient.

55. Utilisation selon la revendication 54, dans laquelle la cachexie est induite par le TNF alpha humain et le patient est un humain.

56. Utilisation selon les revendications 54 et 55 dans laquelle le groupe effecteur est un Fc.

57. Utilisation selon l'une quelconque des revendications 54 à 56 dans laquelle le groupe effecteur - dAb est administré dans une plage de dosages allant de 0,5 à 20 mg/kg.

58. Utilisation selon la revendication 57, dans laquelle le groupe effecteur - dAb est administré dans une plage de dosages allant de 1 à 10 mg/kg.

59. Composition selon la revendication 34 ayant la structure décrite sur la figure 1(g)(ii), dans laquelle les domaines variables simples V_{H} et V_{L} forment chacun un site respectif de liaison à l'épitope ou à l'antigène et la spécificité à l'épitope ou à l'antigène des domaines variables simples est différente.
